# EUROPEAN PATENT APPLICATION

(11) **EP 3 816 296 A1**
(43) Date of publication of application: **05.05.2021**
(21) Application number: 18889963.7
(22) Date of filing: 25.07.2018
(51) Int. Cl.: C12N 15/90, C12N 15/113

(54) **REAGENT AND METHOD FOR REPAIRING FBN1T7498C MUTATION USING BASE EDITING**

(30) Priority: 01.06.2018 CN 201810560722
(71) Applicant: Shanghaitech University, Shanghai 201210 (CN)
(72) Inventor: HUANG, Xingxu, Shanghai, 201210 (CN); LI, Guanglei, Shanghai, 201210 (CN); LI, Jianan, Shanghai, 201210 (CN)
(74) Representative: Cabinet Beau de Loménie
(86) International application number: PCT/CN2018/096968
(87) International publication number: WO 2019/227640

(57) **Abstract**

The present invention provides a reagent and a method for repairing an *FBN1^{T7498C}* mutation by using base editing. A kit for efficiently repairing the *FBN1^{T7498C}* mutation is characterized by comprising a base editor and a repair re-sgRNA directed to the *FBN1^{T7498C}* site. In the present invention, a base editing technology is used to repair the mutation of *FBN1^{T7498C}* through a precise CT single base mutation, thereby providing an efficient and safe method for treating a Marfan syndrome caused by such mutation.

SEQUENCE LISTING

<110> 上海科技大学

<120>利用碱基编辑修复 FBN1T7498C 突变的试剂和方法

<160> 57

<170>PatentIn version 3.5

<210> 1
<211> 23
<212> DNA
<213> Artificial Sequence

<400> 1
cgccaatggt gttaacacat agg 23

<210> 2
<211> 110
<212> DNA
<213> Artificial Sequence

<400> 2
gacgtatggt gttgggtaaa tccgggagga catttgcatg tgaagccgcc aatggtgtta 60
acacgtagga actggcagtt gtgttgcttg gttgcacact catcaagatc 110

<210> 3
<211> 23
<212> DNA
<213> Artificial Sequence

<400> 3

ccgccaatgg tgttaacacg tag 23
<210> 4
<211> 687
<212> DNA
<213> Artificial Sequence

<400> 4

atgagctcag agactggccc agtggctgtg gaccccacat tgagacggcg gatcgagccc 60
catgagtttg aggtattctt cgatccgaga gagctccgca aggagacctg cctgctttac 120
gaaattaatt gggggggccg gcactccatt tggcgacata catcacagaa cactaacaag 180
cacgtcgaag tcaacttcat cgagaagttc acgacagaaa gatatttctg tccgaacaca 240
aggtgcagca ttacctggtt tctcagctac agcccatgcg gcgaatgtag tagggccatc 300
actgaattcc tgtcaaggta tccccacgtc actctgttta tttacatcgc aaggctgtac 360
caccacgctg accccgagaa tcgacaaggc ctgcgggatt tgatctcttc aggtgtgact 420
atccaaatta tgactgagca ggagtcagga tactgctgga gaaactttgt gaattatagc 480
ccgagtaatg aagcccactg gcctaggtat ccccatctgt gggtacgact gtacgttctt 540
gaactgtact gcatcatact gggcctgcct ccttgtctca acattctgag aaggaagcag 600
ccacagctga cattctttac catcgctctt cagtcttgtc attaccagcg actgccccca 660
cacattctct gggccaccgg gttgaaa 687

<210> 5
<211> 687

<212>  DNA
<213>  Artificial Sequence

<400>  5

atgagctcag agactggccc agtggctgtg gaccccacat tgagacggcg gatcgagccc  60
catgagtttg aggtattctt cgatccgaga gagctccgca aggagacctg cctgctttac  120
gaaattaatt gggggggccg gcactccatt tggcgacata catcacagaa cactaacaag  180
cacgtcgaag tcaacttcat cgagaagttc acgacagaaa gatatttctg tccgaacaca  240
aggtgcagca ttacctggtt tctcagctat agcccatgcg gcgaatgtag tagggccatc  300
actgaattcc tgtcaaggta tccccacgtc actctgttta tttacatcgc aaggctgtac  360
caccacgctg acccccgcaa tcgacaaggc ctggaagatt tgatctcttc aggtgtgact  420
atccaaatta tgactgagca ggagtcagga tactgctgga gaaactttgt gaattatagc  480
ccgagtaatg aagcccactg gcctaggtat ccccatctgt gggtacgact gtacgttctt  540
gaactgtact gcatcatact gggcctgcct ccttgtctca acattctgag aaggaagcag  600
ccacagctga cattctttac catcgctctt cagtcttgtc attaccagcg actgccccca  660
cacattctct gggccaccgg gttgaaa  687


<210>  6
<211>  687
<212>  DNA
<213>  Artificial Sequence

<400>  6

atgagctcag agactggccc agtggctgtg gaccccacat tgagacggcg gatcgagccc  60
catgagtttg aggtattctt cgatccgaga gagctccgca aggagacctg cctgctttac  120
gaaattaatt gggggggccg gcactccatt tggcgacata catcacagaa cactaacaag  180
cacgtcgaag tcaacttcat cgagaagttc acgacagaaa gatatttctg tccgaacaca  240
aggtgcagca ttacctggtt tctcagctac agcccatgcg gcgaatgtag tagggccatc  300
actgaattcc tgtcaaggta tccccacgtc actctgttta tttacatcgc aaggctgtac  360
caccacgctg accccgagaa tcgacaaggc ctggaggatt tgatctcttc aggtgtgact  420
atccaaatta tgactgagca ggagtcagga tactgctgga gaaactttgt gaattatagc  480
ccgagtaatg aagcccactg gcctaggtat ccccatctgt gggtacgact gtacgttctt  540
gaactgtact gcatcatact gggcctgcct ccttgtctca acattctgag aaggaagcag  600
ccacagctga cattctttac catcgctctt cagtcttgtc attaccagcg actgccccca  660
cacattctct gggccaccgg gttgaaa  687

<210>  7
<211>  8532
<212>  DNA
<213>  Artificial Sequence

<400>  7

atatgccaag tacgccccct attgacgtca atgacggtaa atggcccgcc tggcattatg  60
cccagtacat gaccttatgg gactttccta cttggcagta catctacgta ttagtcatcg  120
ctattaccat ggtgatgcgg ttttggcagt acatcaatgg gcgtggatag cggtttgact  180
cacggggatt tccaagtctc caccccattg acgtcaatgg gagtttgttt tggcaccaaa  240
atcaacggga ctttccaaaa tgtcgtaaca actccgcccc attgacgcaa atgggcggta  300
ggcgtgtacg gtgggaggtc tatataagca gagctggttt agtgaaccgt cagatccgct  360
agagatccgc ggccgctaat acgactcact atagggagag ccgccaccat gagctcagag  420
actggcccag tggctgtgga ccccacattg agacggcgga tcgagcccca tgagtttgag  480
gtattcttcg atccgagaga gctccgcaag gagacctgcc tgctttacga aattaattgg  540
gggggccggc actccatttg gcgacataca tcacagaaca ctaacaagca cgtcgaagtc  600
aacttcatcg agaagttcac gacagaaaga tatttctgtc cgaacacaag gtgcagcatt  660
acctggtttc tcagctacag cccatgcggc gaatgtagta gggccatcac tgaattcctg  720

tcaaggtatc cccacgtcac tctgtttatt tacatcgcaa ggctgtacca ccacgctgac 780
cccgagaatc gacaaggcct gcgggatttg atctcttcag gtgtgactat ccaaattatg 840
actgagcagg agtcaggata ctgctggaga aactttgtga attatagccc gagtaatgaa 900
gcccactggc ctaggtatcc ccatctgtgg gtacgactgt acgttcttga actgtactgc 960
atcatactgg gcctgcctcc ttgtctcaac attctgagaa ggaagcagcc acagctgaca 1020
ttctttacca tcgctcttca gtcttgtcat taccagcgac tgcccccaca cattctctgg 1080
gccaccgggt tgaaaagcgg cagcgagact cccgggacct cagagtccgc cacacccgaa 1140
agtgataaaa agtattctat tggtttagcc atcggcacta attccgttgg atgggctgtc 1200
ataaccgatg aatacaaagt accttcaaag aaatttaagg tgttggggaa cacagaccgt 1260
cattcgatta aaaagaatct tatcggtgcc ctcctattcg atagtggcga aacggcagag 1320
gcgactcgcc tgaaacgaac cgctcggaga aggtatacac gtcgcaagaa ccgaatatgt 1380
tacttacaag aaatttttag caatgagatg gccaaagttg acgattcttt ctttcaccgt 1440
ttggaagagt ccttccttgt cgaagaggac aagaaacatg aacggcaccc catctttgga 1500
aacatagtag atgaggtggc atatcatgaa aagtacccaa cgatttatca cctcagaaaa 1560
aagctagttg actcaactga taaagcggac ctgaggttaa tctacttggc tcttgcccat 1620
atgataaagt tccgtgggca ctttctcatt gagggtgatc taaatccgga caactcggat 1680
gtcgacaaac tgttcatcca gttagtacaa acctataatc agttgtttga agagaaccct 1740
ataaatgcaa gtggcgtgga tgcgaaggct attcttagcg cccgcctctc taaatcccga 1800
cggctagaaa acctgatcgc acaattaccc ggagagaaga aaaatgggtt gttcggtaac 1860
cttatagcgc tctcactagg cctgacacca aattttaagt cgaacttcga cttagctgaa 1920
gatgccaaat tgcagcttag taaggacacg tacgatgacg atctcgacaa tctactggca 1980
caaattggag atcagtatgc ggacttattt ttggctgcca aaaaccttag cgatgcaatc 2040
ctcctatctg acatactgag agttaatact gagattacca aggcgccgtt atccgcttca 2100
atgatcaaaa ggtacgatga acatcaccaa gacttgacac ttctcaaggc cctagtccgt 2160
cagcaactgc ctgagaaata taaggaaata ttctttgatc agtcgaaaaa cgggtacgca 2220
ggttatattg acggcggagc gagtcaagag gaattctaca agtttatcaa acccatatta 2280
gagaagatgg atgggacgga agagttgctt gtaaaactca atcgcgaaga tctactgcga 2340
aagcagcgga ctttcgacaa cggtagcatt ccacatcaaa tccacttagg cgaattgcat 2400
gctatactta gaaggcagga ggatttttat ccgttcctca aagacaatcg tgaaaagatt 2460
gagaaaatcc taacctttcg cataccttac tatgtgggac ccctggcccg agggaactct 2520
cggttcgcat ggatgacaag aaagtccgaa gaaacgatta ctccatggaa ttttgaggaa 2580
gttgtcgata aaggtgcgtc agctcaatcg ttcatcgaga ggatgaccaa ctttgacaag 2640
aatttaccga acgaaaaagt attgcctaag cacagtttac tttacgagta tttcacagtg 2700
tacaatgaac tcacgaaagt taagtatgtc actgagggca tgcgtaaacc cgcctttcta 2760
agcggagaac agaagaaagc aatagtagat ctgttattca agaccaaccg caaagtgaca 2820
gttaagcaat tgaaagagga ctactttaag aaaattgaat gcttcgattc tgtcgagatc 2880
tccggggtag aagatcgatt taatgcgtca cttggtacgt atcatgacct cctaaagata 2940
attaaagata aggacttcct ggataacgaa gagaatgaag atatcttaga agatatagtg 3000
ttgactctta ccctctttga agatcgggaa atgattgagg aaagactaaa aacatacgct 3060
cacctgttcg acgataaggt tatgaaacag ttaaagaggc gtcgctatac gggctgggga 3120
cgattgtcgc ggaaacttat caacgggata agagacaagc aaagtggtaa aactattctc 3180
gattttctaa agagcgacgg cttcgccaat aggaacttta tgcagctgat ccatgatgac 3240
tctttaacct tcaaagagga tatacaaaag gcacaggttt ccggacaagg ggactcattg 3300
cacgaacata ttgcgaatct tgctggttcg ccagccatca aaaagggcat actccagaca 3360
gtcaaagtag tggatgagct agttaaggtc atgggacgtc acaaaccgga aaacattgta 3420
atcgagatgg cacgcgaaaa tcaaacgact cagaaggggc aaaaaaacag tcgagagcgg 3480
atgaagagaa tagaagaggg tattaaagaa ctgggcagcc agatcttaaa ggagcatcct 3540
gtggaaaata cccaattgca gaacgagaaa ctttacctct attacctaca aaatggaagg 3600
gacatgtatg ttgatcagga actggacata aaccgtttat ctgattacga cgtcgatcac 3660
attgtacccc aatccttttt gaaggacgat tcaatcgaca ataaagtgct tacacgctcg 3720
gataagaacc gagggaaaag tgacaatgtt ccaagcgagg aagtcgtaaa gaaaatgaag 3780
aactattggc ggcagctcct aaatgcgaaa ctgataacgc aaagaaagtt cgataactta 3840
actaaagctg agaggggtgg cttgtctgaa cttgacaagg ccggatttat taaacgtcag 3900
ctcgtggaaa cccgccaaat cacaaagcat gttgcacaga tactagattc ccgaatgaat 3960
acgaaatacg acgagaacga taagctgatt cgggaagtca aagtaatcac tttaaagtca 4020
aaattggtgt cggacttcag aaaggatttt caattctata aagttaggga gataaataac 4080
taccaccatg cgcacgacgc ttatcttaat gccgtcgtag ggaccgcact cattaagaaa 4140
tacccgaagc tagaaagtga gtttgtgtat ggtgattaca aagtttatga cgtccgtaag 4200

atgatcgcga aaagcgaaca ggagataggc aaggctacag ccaaatactt cttttattct 4260
aacattatga atttctttaa gacggaaatc actctggcaa acggagagat acgcaaacga 4320
cctttaattg aaaccaatgg ggagacaggt gaaatcgtat gggataaggg ccgggacttc 4380
gcgacggtga gaaaagtttt gtccatgccc caagtcaaca tagtaaagaa aactgaggtg 4440
cagaccggag ggttttcaaa ggaatcgatt cttccaaaaa ggaatagtga taagctcatc 4500
gctcgtaaaa aggactggga cccgaaaaag tacggtggct tcgatagccc tacagttgcc 4560
tattctgtcc tagtagtggc aaaagttgag aagggaaaat ccaagaaact gaagtcagtc 4620
aaagaattat tggggataac gattatggag cgctcgtctt ttgaaaagaa ccccatcgac 4680
ttccttgagg cgaaaggtta caaggaagta aaaaaggatc tcataattaa actaccaaag 4740
tatagtctgt ttgagttaga aaatggccga aaacggatgt tggctagcgc cggagagctt 4800
caaaagggga acgaactcgc actaccgtct aaatacgtga atttcctgta tttagcgtcc 4860
cattacgaga agttgaaagg ttcacctgaa gataacgaac agaagcaact ttttgttgag 4920
cagcacaaac attatctcga cgaaatcata gagcaaattt cggaattcag taagagagtc 4980
atcctagctg atgccaatct ggacaaagta ttaagcgcat acaacaagca cagggataaa 5040
cccatacgtg agcaggcgga aaatattatc catttgttta ctcttaccaa cctcggcgct 5100
ccagccgcat tcaagtattt tgacacaacg atagatcgca aacgatacac ttctaccaag 5160
gaggtgctag acgcgacact gattcaccaa tccatcacgg gattatatga aactcggata 5220
gatttgtcac agcttggggg tgactctggt ggttctacta atctgtcaga tattattgaa 5280
aaggagaccg gtaagcaact ggttatccag gaatccatcc tcatgctccc agaggaggtg 5340
gaagaagtca ttgggaacaa gccggaaagc gatatactcg tgcacaccgc ctacgacgag 5400
agcaccgacg agaatgtcat gcttctgact agcgacgccc ctgaatacaa gccttgggct 5460
ctggtcatac aggatagcaa cggtgagaac aagattaaga tgctctctgg tggttctccc 5520
aagaagaaga ggaaagtcta accggtcatc atcaccatca ccattgagtt taaacccgct 5580
gatcagcctc gactgtgcct tctagttgcc agccatctgt tgtttgcccc tcccccgtgc 5640
cttccttgac cctggaaggt gccactccca ctgtcctttc ctaataaaat gaggaaattg 5700
catcgcattg tctgagtagg tgtcattcta ttctgggggg tggggtgggg caggacagca 5760
agggggagga ttgggaagac aatagcaggc atgctgggga tgcggtgggc tctatggctt 5820
ctgaggcgga aagaaccagc tggggctcga taccgtcgac ctctagctag agcttggcgt 5880
aatcatggtc atagctgttt cctgtgtgaa attgttatcc gctcacaatt ccacacaaca 5940
tacgagccgg aagcataaag tgtaaagcct agggtgccta atgagtgagc taactcacat 6000
taattgcgtt gcgctcactg cccgctttcc agtcgggaaa cctgtcgtgc cagctgcatt 6060
aatgaatcgg ccaacgcgcg gggagaggcg gtttgcgtat tgggcgctct tccgcttcct 6120
cgctcactga ctcgctgcgc tcggtcgttc ggctgcggcg agcggtatca gctcactcaa 6180
aggcggtaat acggttatcc acagaatcag gggataacgc aggaaagaac atgtgagcaa 6240
aaggccagca aaaggccagg aaccgtaaaa aggccgcgtt gctggcgttt ttccataggc 6300
tccgcccccc tgacgagcat cacaaaaatc gacgctcaag tcagaggtgg cgaaacccga 6360
caggactata aagataccag gcgtttcccc ctggaagctc cctcgtgcgc tctcctgttc 6420
cgaccctgcc gcttaccgga tacctgtccg cctttctccc ttcgggaagc gtggcgcttt 6480
ctcatagctc acgctgtagg tatctcagtt cggtgtaggt cgttcgctcc aagctgggct 6540
gtgtgcacga accccccgtt cagcccgacc gctgcgcctt atccggtaac tatcgtcttg 6600
agtccaaccc ggtaagacac gacttatcgc cactggcagc agccactggt aacaggatta 6660
gcagagcgag gtatgtaggc ggtgctacag agttcttgaa gtggtggcct aactacggct 6720
acactagaag aacagtattt ggtatctgcg ctctgctgaa gccagttacc ttcggaaaaa 6780
gagttggtag ctcttgatcc ggcaaacaaa ccaccgctgg tagcggtggt ttttttgttt 6840
gcaagcagca gattacgcgc agaaaaaaag gatctcaaga agatcctttg atcttttcta 6900
cggggtctga cgctcagtgg aacgaaaact cacgttaagg gattttggtc atgagattat 6960
caaaaaggat cttcacctag atccttttaa attaaaaatg aagttttaaa tcaatctaaa 7020
gtatatatga gtaaacttgg tctgacagtt accaatgctt aatcagtgag gcacctatct 7080
cagcgatctg tctatttcgt tcatccatag ttgcctgact ccccgtcgtg tagataacta 7140
cgatacggga gggcttacca tctggcccca gtgctgcaat gataccgcga gacccacgct 7200
caccggctcc agatttatca gcaataaacc agccagccgg aagggccgag cgcagaagtg 7260
gtcctgcaac tttatccgcc tccatccagt ctattaattg ttgccgggaa gctagagtaa 7320
gtagttcgcc agttaatagt ttgcgcaacg ttgttgccat tgctacaggc atcgtggtgt 7380
cacgctcgtc gtttggtatg gcttcattca gctccggttc ccaacgatca aggcgagtta 7440
catgatcccc catgttgtgc aaaaaagcgg ttagctcctt cggtcctccg atcgttgtca 7500
gaagtaagtt ggccgcagtg ttatcactca tggttatggc agcactgcat aattctctta 7560
ctgtcatgcc atccgtaaga tgcttttctg tgactggtga gtactcaacc aagtcattct 7620
gagaatagtg tatgcggcga ccgagttgct cttgcccggc gtcaatacgg gataataccg 7680

cgccacatag cagaacttta aaagtgctca tcattggaaa acgttcttcg gggcgaaaac 7740
tctcaaggat cttaccgctg ttgagatcca gttcgatgta acccactcgt gcacccaact 7800
gatcttcagc atcttttact ttcaccagcg tttctgggtg agcaaaaaca ggaaggcaaa 7860
atgccgcaaa aaagggaata agggcgacac ggaaatgttg aatactcata ctcttccttt 7920
ttcaatatta ttgaagcatt tatcagggtt attgtctcat gagcggatac atatttgaat 7980
gtatttagaa aaataaacaa ataggggttc cgcgcacatt tccccgaaaa gtgccacctg 8040
acgtcgacgg atcgggagat cgatctcccg atcccctagg gtcgactctc agtacaatct 8100
gctctgatgc cgcatagtta agccagtatc tgctccctgc ttgtgtgttg gaggtcgctg 8160
agtagtgcgc gagcaaaatt taagctacaa caaggcaagg cttgaccgac aattgcatga 8220
agaatctgct tagggttagg cgttttgcgc tgcttcgcga tgtacgggcc agatatacgc 8280
gttgacattg attattgact agttattaat agtaatcaat tacggggtca ttagttcata 8340
gcccatatat ggagttccgc gttacataac ttacggtaaa tggcccgcct ggctgaccgc 8400
ccaacgaccc ccgcccattg acgtcaataa tgacgtatgt tcccatagta acgccaatag 8460
ggactttcca ttgacgtcaa tgggtggagt atttacggta aactgcccac ttggcagtac 8520
atcaagtgta tc 8532

<210> 8
<211> 8532
<212> DNA
<213> Artificial Sequence

<400> 8
   atatgccaag tacgccccct attgacgtca atgacggtaa atggcccgcc tggcattatg 60
   cccagtacat gaccttatgg gactttccta cttggcagta catctacgta ttagtcatcg 120
   ctattaccat ggtgatgcgg ttttggcagt acatcaatgg gcgtggatag cggtttgact 180
   cacggggatt tccaagtctc caccccattg acgtcaatgg gagtttgttt tggcaccaaa 240
   atcaacggga ctttccaaaa tgtcgtaaca actccgcccc attgacgcaa atgggcggta 300
   ggcgtgtacg gtgggaggtc tatataagca gagctggttt agtgaaccgt cagatccgct 360
   agagatccgc ggccgctaat acgactcact atagggagag ccgccaccat gagctcagag 420
   actggcccag tggctgtgga ccccacattg agacggcgga tcgagcccca tgagtttgag 480
   gtattcttcg atccgagaga gctccgcaag gagacctgcc tgctttacga aattaattgg 540
   gggggccggc actccatttg gcgacataca tcacagaaca ctaacaagca cgtcgaagtc 600
   aacttcatcg agaagttcac gacagaaaga tatttctgtc cgaacacaag gtgcagcatt 660
   acctggtttc tcagctatag cccatgcggc gaatgtagta gggccatcac tgaattcctg 720
   tcaaggtatc cccacgtcac tctgtttatt tacatcgcaa ggctgtacca ccacgctgac 780
   ccccgcaatc gacaaggcct ggaagatttg atctcttcag gtgtgactat ccaaattatg 840
   actgagcagg agtcaggata ctgctggaga aactttgtga attatagccc gagtaatgaa 900
   gcccactggc ctaggtatcc ccatctgtgg gtacgactgt acgttcttga actgtactgc 960
   atcatactgg gcctgcctcc ttgtctcaac attctgagaa ggaagcagcc acagctgaca 1020
   ttctttacca tcgctcttca gtcttgtcat taccagcgac tgcccccaca cattctctgg 1080
   gccaccgcgt tgaaaagcgg cagcgagact cccgggacct cagagtccgc cacacccgaa 1140
   agtgataaaa agtattctat tggtttagcc atcggcacta attccgttgg atgggctgtc 1200
   ataaccgatg aatacaaagt accttcaaag aaatttaagg tgttggggaa cacagaccgt 1260
   cattcgatta aaaagaatct tatcggtgcc ctcctattcg atagtggcga aacggcagag 1320
   gcgactcgcc tgaaacgaac cgctcggaga aggtatacac gtcgcaagaa ccgaatatgt 1380
   tacttacaag aaatttttag caatgagatg gccaaagttg acgattcttt ctttcaccgt 1440
   ttggaagagt ccttccttgt cgaagaggac aagaaacatg aacggcaccc catctttgga 1500
   aacatagtag atgaggtggc atatcatgaa aagtacccaa cgatttatca cctcagaaaa 1560
   aagctagttg actcaactga taaagcggac ctgaggttaa tctacttggc tcttgcccat 1620
   atgataaagt tccgtgggca ctttctcatt gagggtgatc taaatccgga caactcggat 1680
   gtcgacaaac tgttcatcca gttagtacaa acctataatc agttgtttga agagaaccct 1740
   ataaatgcaa gtggcgtgga tgcgaaggct attcttagcg cccgcctctc taaatcccga 1800
   cggctagaaa acctgatcgc acaattaccc ggagagaaga aaaatgggtt gttcggtaac 1860
   cttatagcgc tctcactagg cctgacacca aattttaagt cgaacttcga cttagctgaa 1920
   gatgccaaat tgcagcttag taaggacacg tacgatgacg atctcgacaa tctactggca 1980
   caaattggag atcagtatgc ggacttattt ttggctgcca aaaaccttag cgatgcaatc 2040
   ctcctatctg acatactgag agttaatact gagattacca aggcgccgtt atccgcttca 2100
   atgatcaaaa ggtacgatga acatcaccaa gacttgacac ttctcaaggc cctagtccgt 2160
   cagcaactgc ctgagaaata taaggaaata ttctttgatc agtcgaaaaa cgggtacgca 2220
   ggttatattg acggcggagc gagtcaagag gaattctaca agtttatcaa acccatatta 2280
   gagaagatgg atgggacgga agagttgctt gtaaaactca atcgcgaaga tctactgcga 2340
   aagcagcgga ctttcgacaa cggtagcatt ccacatcaaa tccacttagg cgaattgcat 2400
   gctatactta gaaggcagga ggatttttat ccgttcctca aagacaatcg tgaaaagatt 2460
   gagaaaatcc taacctttcg cataccttac tatgtgggac ccctggcccg agggaactct 2520
   cggttcgcat ggatgacaag aaagtccgaa gaaacgatta ctccatggaa ttttgaggaa 2580
   gttgtcgata aaggtgcgtc agctcaatcg ttcatcgaga ggatgaccaa ctttgacaag 2640
   aatttaccga acgaaaaagt attgcctaag cacagtttac tttacgagta tttcacagtg 2700
   tacaatgaac tcacgaaagt taagtatgtc actgaggcca tgcgtaaacc cgcctttcta 2760
   agcggagaac agaagaaagc aatagtagat ctgttattca agaccaaccg caaagtgaca 2820
   gttaagcaat tgaaagagga ctactttaag aaaattgaat gcttcgattc tgtcgagatc 2880
   tccggggtag aagatcgatt taatgcgtca cttggtacgt atcatgacct cctaaagata 2940
   attaaagata aggacttcct ggataacgaa gagaatgaag atatcttaga agatatagtg 3000
   ttgactctta ccctctttga agatcgggaa atgattgagg aaagactaaa aacatacgct 3060
   cacctgttcg acgataaggt tatgaaacag ttaaagaggc gtcgctatac gggctgggga 3120
   cgattgtcgc ggaaacttat caacgggata agagacaagc aaagtggtaa aactattctc 3180
   gattttctaa agagcgacgg cttcgccaat aggaacttta tgcagctgat ccatgatgac 3240
   tctttaacct tcaaagagga tatacaaaag gcacaggttt ccggacaagg ggactcattg 3300
   cacgaacata ttgcgaatct tgctggttcg ccagccatca aaaagggcat actccagaca 3360
   gtcaaagtag tggatgagct agttaaggtc atgggacgtc acaaaccgga aaacattgta 3420
   atcgagatgg cacgcgaaaa tcaaacgact cagaaggggc aaaaaaacag tcgagagcgg 3480
   atgaagagaa tagaagaggg tattaaagaa ctgggcagcc agatcttaaa ggagcatcct 3540
   gtggaaaata cccaattgca gaacgagaaa ctttacctct attacctaca aaatggaagg 3600
   gacatgtatg ttgatcagga actggacata aaccgtttat ctgattacga cgtcgatcac 3660
   attgtacccc aatccttttt gaaggacgat tcaatcgaca ataaagtgct tacacgctcg 3720
   gataagaacc gagggaaaag tgacaatgtt ccaagcgagg aagtcgtaaa gaaaatgaag 3780
   aactattggc ggcagctcct aaatgcgaaa ctgataacgc aaagaaagtt cgataactta 3840
   actaaagctg agaggggtgg cttgtctgaa cttgacaagg ccggatttat taaacgtcag 3900
   ctcgtggaaa cccgccaaat cacaaagcat gttgcacaga tactagattc ccgaatgaat 3960
   acgaaatacg acgagaacga taagctgatt cgggaagtca aagtaatcac tttaaagtca 4020
   aaattggtgt cggacttcag aaaggatttt caattctata aagttaggga gataaataac 4080
   taccaccatg cgcacgacgc ttatcttaat gccgtcgtag ggaccgcact cattaagaaa 4140
   tacccgaagc tagaaagtga gtttgtgtat ggtgattaca aagtttatga cgtccgtaag 4200
   atgatcgcga aaagcgaaca ggagataggc aaggctacag ccaaatactt cttttattct 4260
   aacattatga atttctttaa gacggaaatc actctggcaa acggagagat acgcaaacga 4320
   cctttaattg aaaccaatgg ggagacaggt gaaatcgtat gggataaggg ccgggacttc 4380
   gcgacggtga gaaaagtttt gtccatgccc caagtcaaca tagtaaagaa aactgaggtg 4440
   cagaccggag ggttttcaaa ggaatcgatt cttccaaaaa ggaatagtga taagctcatc 4500
   gctcgtaaaa aggactggga cccgaaaaag tacggtggct tcgatagccc tacagttgcc 4560
   tattctgtcc tagtagtggc aaaagttgag aagggaaaat ccaagaaact gaagtcagtc 4620
   aaagaattat tggggataac gattatggag cgctcgtctt ttgaaaagaa ccccatcgac 4680
   ttccttgagg cgaaaggtta caaggaagta aaaaaggatc tcataattaa actaccaaag 4740
   tatagtctgt ttgagttaga aaatggccga aaacggatgt tggctagcgc cggagagctt 4800
   caaaagggga acgaactcgc actaccgtct aaatacgtga atttcctgta tttagcgtcc 4860
   cattacgaga agttgaaagg ttcacctgaa gataacgaac agaagcaact ttttgttgag 4920
   cagcacaaac attatctcga cgaaatcata gagcaaattt cggaattcag taagagagtc 4980
   atcctagctg atgccaatct ggacaaagta ttaagcgcat acaacaagca cagggataaa 5040
   cccatacgtg agcaggcgga aaatattatc catttgttta ctcttaccaa cctcggcgct 5100
   ccagccgcat tcaagtattt tgacacaacg atagatcgca aacgatacac ttctaccaag 5160
   gaggtgctag acgcgacact gattcaccaa tccatcacgg gattatatga aactcggata 5220
   gatttgtcac agcttggggg tgactctggt ggttctacta atctgtcaga tattattgaa 5280
   aaggagaccg gtaagcaact ggttatccag gaatccatcc tcatgctccc agaggaggtg 5340
   gaagaagtca ttgggaacaa gccggaaagc gatatactcg tgcacaccgc ctacgacgag 5400
   agcaccgacg agaatgtcat gcttctgact agcgacgccc ctgaatacaa gccttgggct 5460
   ctggtcatac aggatagcaa cggtgagaac aagattaaga tgctctctgg tggttctccc 5520
   aagaagaaga ggaaagtcta accggtcatc atcaccatca ccattgagtt taaacccgct 5580
   gatcagcctc gactgtgcct tctagttgcc agccatctgt tgtttgcccc tcccccgtgc 5640
   cttccttgac cctggaaggt gccactccca ctgtcctttc ctaataaaat gaggaaattg 5700
   catcgcattg tctgagtagg tgtcattcta ttctgggggg tggggtgggg caggacagca 5760
   agggggagga ttgggaagac aatagcaggc atgctgggga tgcggtgggc tctatggctt 5820
   ctgaggcgga aagaaccagc tggggctcga taccgtcgac ctctagctag agcttggcgt 5880
   aatcatggtc atagctgttt cctgtgtgaa attgttatcc gctcacaatt ccacacaaca 5940
   tacgagccgg aagcataaag tgtaaagcct agggtgccta atgagtgagc taactcacat 6000
   taattgcgtt gcgctcactg cccgctttcc agtcgggaaa cctgtcgtgc cagctgcatt 6060
   aatgaatcgg ccaacgcgcg gggagaggcg gtttgcgtat tgggcgctct tccgcttcct 6120
   cgctcactga ctcgctgcgc tcggtcgttc ggctgcggcg agcggtatca gctcactcaa 6180
   aggcggtaat acggttatcc acagaatcag gggataacgc aggaaagaac atgtgagcaa 6240
   aaggccagca aaaggccagg aaccgtaaaa aggccgcgtt gctggcgttt ttccataggc 6300
   tccgcccccc tgacgagcat cacaaaaatc gacgctcaag tcagaggtgg cgaaacccga 6360
   caggactata aagataccag gcgtttcccc ctggaagctc cctcgtgcgc tctcctgttc 6420
   cgaccctgcc gcttaccgga tacctgtccg cctttctccc ttcgggaagc gtggcgcttt 6480
   ctcatagctc acgctgtagg tatctcagtt cggtgtaggt cgttcgctcc aagctgggct 6540
   gtgtgcacga accccccgtt cagcccgacc gctgcgcctt atccggtaac tatcgtcttg 6600
   agtccaaccc ggtaagacac gacttatcgc cactggcagc agccactggt aacaggatta 6660
   gcagagcgag gtatgtaggc ggtgctacag agttcttgaa gtggtggcct aactacggct 6720
   acactagaag aacagtattt ggtatctgcg ctctgctgaa gccagttacc ttcggaaaaa 6780
   gagttggtag ctcttgatcc ggcaaacaaa ccaccgctgg tagcggtggt ttttttgttt 6840
   gcaagcagca gattacgcgc agaaaaaaag gatctcaaga agatcctttg atcttttcta 6900
   cggggtctga cgctcagtgg aacgaaaact cacgttaagg gattttggtc atgagattat 6960
   caaaaaggat cttcacctag atccttttaa attaaaaatg aagttttaaa tcaatctaaa 7020
   gtatatatga gtaaacttgg tctgacagtt accaatgctt aatcagtgag gcacctatct 7080
   cagcgatctg tctatttcgt tcatccatag ttgcctgact ccccgtcgtg tagataacta 7140
   cgatacggga gggcttacca tctggcccca gtgctgcaat gataccgcga gacccacgct 7200
   caccggctcc agatttatca gcaataaacc agccagccgg aagggccgag cgcagaagtg 7260
   gtcctgcaac tttatccgcc tccatccagt ctattaattg ttgccgggaa gctagagtaa 7320
   gtagttcgcc agttaatagt ttgcgcaacg ttgttgccat tgctacaggc atcgtggtgt 7380
   cacgctcgtc gtttggtatg gcttcattca gctccggttc ccaacgatca aggcgagtta 7440
   catgatcccc catgttgtgc aaaaaagcgg ttagctcctt cggtcctccg atcgttgtca 7500
   gaagtaagtt ggccgcagtg ttatcactca tggttatggc agcactgcat aattctctta 7560
   ctgtcatgcc atccgtaaga tgcttttctg tgactggtga gtactcaacc aagtcattct 7620
   gagaatagtg tatgcggcga ccgagttgct cttgcccggc gtcaatacgg gataataccg 7680
   cgccacatag cagaacttta aaagtgctca tcattggaaa acgttcttcg gggcgaaaac 7740
   tctcaaggat cttaccgctg ttgagatcca gttcgatgta acccactcgt gcacccaact 7800
   gatcttcagc atcttttact ttcaccagcg tttctgggtg agcaaaaaca ggaaggcaaa 7860
   atgccgcaaa aaagggaata agggcgacac ggaaatgttg aatactcata ctcttccttt 7920
   ttcaatatta ttgaagcatt tatcagggtt attgtctcat gagcggatac atatttgaat 7980
   gtatttagaa aaataaacaa ataggggttc cgcgcacatt tccccgaaaa gtgccacctg 8040
   acgtcgacgg atcgggagat cgatctcccg atcccctagg gtcgactctc agtacaatct 8100
   gctctgatgc cgcatagtta agccagtatc tgctccctgc ttgtgtgttg gaggtcgctg 8160
   agtagtgcgc gagcaaaatt taagctacaa caaggcaagg cttgaccgac aattgcatga 8220
   agaatctgct tagggttagg cgttttgcgc tgcttcgcga tgtacgggcc agatatacgc 8280
   gttgacattg attattgact agttattaat agtaatcaat tacggggtca ttagttcata 8340
   gcccatatat ggagttccgc gttacataac ttacggtaaa tggcccgcct ggctgaccgc 8400
   ccaacgaccc ccgcccattg acgtcaataa tgacgtatgt tcccatagta acgccaatag 8460
   ggactttcca ttgacgtcaa tgggtggagt atttacggta aactgcccac ttggcagtac 8520
   atcaagtgta tc 8532

<210> 9
<211> 8604
<212> DNA
<213> Artificial Sequence

<400> 9

atatgccaag tacgccccct attgacgtca atgacggtaa atggcccgcc tggcattatg 60
cccagtacat gaccttatgg gactttccta cttggcagta catctacgta ttagtcatcg 120
ctattaccat ggtgatgcgg ttttggcagt acatcaatgg gcgtggatag cggtttgact 180
cacggggatt tccaagtctc caccccattg acgtcaatgg gagtttgttt tggcaccaaa 240
atcaacggga ctttccaaaa tgtcgtaaca actccgcccc attgacgcaa atgggcggta 300
ggcgtgtacg gtgggaggtc tatataagca gagctggttt agtgaaccgt cagatccgct 360
agagatccgc ggccgctaat acgactcact atagggagac ccaagctggc tagcaccatg 420
ggacctaaga aaaagaggaa ggtgtctaga gactacaagg atgacgacga taaaggatcc 480
atgagctcag agactggccc agtggctgtg gaccccacat tgagacggcg gatcgagccc 540
catgagtttg aggtattctt cgatccgaga gagctccgca aggagacctg cctgctttac 600
gaaattaatt gggggggccg gcactccatt tggcgacata catcacagaa cactaacaag 660
cacgtcgaag tcaacttcat cgagaagttc acgacagaaa gatatttctg tccgaacaca 720
aggtgcagca ttacctggtt tctcagctac agcccatgcg gcgaatgtag tagggccatc 780
actgaattcc tgtcaaggta tccccacgtc actctgttta tttacatcgc aaggctgtac 840
caccacgctg accccgagaa tcgacaaggc ctggaggatt tgatctcttc aggtgtgact 900
atccaaatta tgactgagca ggagtcagga tactgctgga gaaactttgt gaattatagc 960
ccgagtaatg aagcccactg gcctaggtat ccccatctgt gggtacgact gtacgttctt 1020
gaactgtact gcatcatact gggcctgcct ccttgtctca acattctgag aaggaagcag 1080
ccacagctga cattctttac catcgctctt cagtcttgtc attaccagcg actgccccca 1140
cacattctct gggccaccgg gttgaaaagc ggcagcgaga ctcccgggac ctcagagtcc 1200
gccacacccg aaagtgataa aaagtattct attggtttag ccatcggcac taattccgtt 1260
ggatgggctg tcataaccga tgaatacaaa gtaccttcaa agaaatttaa ggtgttgggg 1320
aacacagacc gtcattcgat taaaaagaat cttatcggtg ccctcctatt cgatagtggc 1380
gaaacggcag aggcgactcg cctgaaacga accgctcgga gaaggtatac acgtcgcaag 1440
aaccgaatat gttacttaca agaaattttt agcaatgaga tggccaaagt tgacgattct 1500
ttctttcacc gtttggaaga gtccttcctt gtcgaagagg acaagaaaca tgaacggcac 1560
cccatctttg gaaacatagt agatgaggtg gcatatcatg aaaagtaccc aacgatttat 1620
cacctcagaa aaaagctagt tgactcaact gataaagcgg acctgaggtt aatctacttg 1680
gctcttgccc atatgataaa gttccgtggg cactttctca ttgagggtga tctaaatccg 1740
gacaactcgg atgtcgacaa actgttcatc cagttagtac aaacctataa tcagttgttt 1800
gaagagaacc ctataaatgc aagtggcgtg gatgcgaagg ctattcttag cgcccgcctc 1860
tctaaatccc gacggctaga aaacctgatc gcacaattac ccggagagaa gaaaaatggg 1920
ttgttcggta accttatagc gctctcacta ggcctgacac caaattttaa gtcgaacttc 1980
gacttagctg aagatgccaa attgcagctt agtaaggaca cgtacgatga cgatctcgac 2040
aatctactgg cacaaattgg agatcagtat gcggacttat ttttggctgc caaaaacctt 2100
agcgatgcaa tcctcctatc tgacatactg agagttaata ctgagattac caaggcgccg 2160
ttatccgctt caatgatcaa aaggtacgat gaacatcacc aagacttgac acttctcaag 2220
gccctagtcc gtcagcaact gcctgagaaa tataaggaaa tattctttga tcagtcgaaa 2280
aacgggtacg caggttatat tgacggcgga gcgagtcaag aggaattcta caagtttatc 2340
aaacccatat tagagaagat ggatgggacg gaagagttgc ttgtaaaact caatcgcgaa 2400
gatctactgc gaaagcagcg gactttcgac aacggtagca ttccacatca aatccactta 2460
ggcgaattgc atgctatact tagaaggcag gaggattttt atccgttcct caaagacaat 2520
cgtgaaaaga ttgagaaaat cctaaccttt cgcatacctt actatgtggg acccctggcc 2580
cgagggaact ctcggttcgc atggatgaca agaaagtccg aagaaacgat tactccatgg 2640
aattttgagg aagttgtcga taaaggtgcg tcagctcaat cgttcatcga gaggatgacc 2700
aactttgaca agaatttacc gaacgaaaaa gtattgccta agcacagttt actttacgag 2760
tatttcacag tgtacaatga actcacgaaa gttaagtatg tcactgaggg catgcgtaaa 2820
cccgcctttc taagcggaga acagaagaaa gcaatagtag atctgttatt caagaccaac 2880
cgcaaagtga cagttaagca attgaaagag gactacttta agaaaattga atgcttcgat 2940
tctgtcgaga tctccggggt agaagatcga tttaatgcgt cacttggtac gtatcatgac 3000
ctcctaaaga taattaaaga taaggacttc ctggataacg aagagaatga agatatctta 3060
gaagatatag tgttgactct taccctcttt gaagatcggg aaatgattga ggaaagacta 3120
aaaacatacg ctcacctgtt cgacgataag gttatgaaac agttaaagag gcgtcgctat 3180
acgggctggg gacgattgtc gcggaaactt atcaacggga taagagacaa gcaaagtggt 3240
aaaactattc tcgattttct aaagagcgac ggcttcgcca ataggaactt tatgcagctg 3300
atccatgatg actctttaac cttcaaagag gatatacaaa aggcacaggt ttccggacaa 3360

ggggactcat tgcacgaaca tattgcgaat cttgctggtt cgccagccat caaaaagggc 3420
atactccaga cagtcaaagt agtggatgag ctagttaagg tcatgggacg tcacaaaccg 3480
gaaaacattg taatcgagat ggcacgcgaa aatcaaacga ctcagaaggg gcaaaaaaac 3540
agtcgagagc ggatgaagag aatagaagag ggtattaaag aactgggcag ccagatctta 3600
aaggagcatc ctgtggaaaa tacccaattg cagaacgaga aactttacct ctattaccta 3660
caaaatggaa gggacatgta tgttgatcag gaactggaca taaaccgttt atctgattac 3720
gacgtcgatc acattgtacc ccaatccttt ttgaaggacg attcaatcga caataaagtg 3780
cttacacgct cggataagaa ccgagggaaa agtgacaatg ttccaagcga ggaagtcgta 3840
aagaaaatga agaactattg gcggcagctc ctaaatgcga aactgataac gcaaagaaag 3900
ttcgataact taactaaagc tgagaggggt ggcttgtctg aacttgacaa ggccggattt 3960
attaaacgtc agctcgtgga aacccgccaa atcacaaagc atgttgcaca gatactagat 4020
tcccgaatga atacgaaata cgacgagaac gataagctga ttcgggaagt caaagtaatc 4080
actttaaagt caaaattggt gtcggacttc agaaaggatt ttcaattcta taaagttagg 4140
gagataaata actaccacca tgcgcacgac gcttatctta atgccgtcgt agggaccgca 4200
ctcattaaga aatacccgaa gctagaaagt gagtttgtgt atggtgatta caaagtttat 4260
gacgtccgta agatgatcgc gaaaagcgaa caggagatag gcaaggctac agccaaatac 4320
ttcttttatt ctaacattat gaatttcttt aagacggaaa tcactctggc aaacggagag 4380
atacgcaaac gacctttaat tgaaaccaat ggggagacag gtgaaatcgt atgggataag 4440
ggccgggact tcgcgacggt gagaaaagtt ttgtccatgc cccaagtcaa catagtaaag 4500
aaaactgagg tgcagaccgg agggttttca aaggaatcga ttcttccaaa aaggaatagt 4560
gataagctca tcgctcgtaa aaaggactgg gacccgaaaa agtacggtgg cttcgatagc 4620
cctacagttg cctattctgt cctagtagtg gcaaaagttg agaagggaaa atccaagaaa 4680
ctgaagtcag tcaaagaatt attggggata acgattatgg agcgctcgtc ttttgaaaag 4740
aaccccatcg acttccttga ggcgaaaggt tacaaggaag taaaaaagga tctcataatt 4800
aaactaccaa agtatagtct gtttgagtta gaaaatggcc gaaaacggat gttggctagc 4860
gccggagagc ttcaaaaggg gaacgaactc gcactaccgt ctaaatacgt gaatttcctg 4920
tatttagcgt cccattacga gaagttgaaa ggttcacctg aagataacga acagaagcaa 4980
ctttttgttg agcagcacaa acattatctc gacgaaatca tagagcaaat ttcggaattc 5040
agtaagagag tcatcctagc tgatgccaat ctggacaaag tattaagcgc atacaacaag 5100
cacagggata aacccatacg tgagcaggcg gaaaatatta tccatttgtt tactcttacc 5160
aacctcggcg ctccagccgc attcaagtat tttgacacaa cgatagatcg caaacgatac 5220
acttctacca aggaggtgct agacgcgaca ctgattcacc aatccatcac gggattatat 5280
gaaactcgga tagatttgtc acagcttggg ggtgactctg gtggttctac taatctgtca 5340
gatattattg aaaaggagac cggtaagcaa ctggttatcc aggaatccat cctcatgctc 5400
ccagaggagg tggaagaagt cattgggaac aagccggaaa gcgatatact cgtgcacacc 5460
gcctacgacg agagcaccga cgagaatgtc atgcttctga ctagcgacgc ccctgaatac 5520
aagccttggg ctctggtcat acaggatagc aacggtgaga acaagattaa gatgctctct 5580
ggtggttctc ccaagaagaa gaggaaagtc taaccggtca tcatcaccat caccattgag 5640
tttaaacccg ctgatcagcc tcgactgtgc cttctagttg ccagccatct gttgtttgcc 5700
cctcccccgt gccttccttg accctggaag gtgccactcc cactgtcctt tcctaataaa 5760
atgaggaaat tgcatcgcat tgtctgagta ggtgtcattc tattctgggg ggtggggtgg 5820
ggcaggacag caagggggag gattgggaag acaatagcag gcatgctggg gatgcggtgg 5880
gctctatggc ttctgaggcg gaaagaacca gctggggctc gataccgtcg acctctagct 5940
agagcttggc gtaatcatgg tcatagctgt ttcctgtgtg aaattgttat ccgctcacaa 6000
ttccacacaa catacgagcc ggaagcataa agtgtaaagc ctagggtgcc taatgagtga 6060
gctaactcac attaattgcg ttgcgctcac tgcccgcttt ccagtcggga aacctgtcgt 6120
gccagctgca ttaatgaatc ggccaacgcg cggggagagg cggtttgcgt attgggcgct 6180
cttccgcttc ctcgctcact gactcgctgc gctcggtcgt tcggctgcgg cgagcggtat 6240
cagctcactc aaaggcggta atacggttat ccacagaatc aggggataac gcaggaaaga 6300
acatgtgagc aaaaggccag caaaaggcca ggaaccgtaa aaaggccgcg ttgctggcgt 6360
ttttccatag gctccgcccc cctgacgagc atcacaaaaa tcgacgctca agtcagaggt 6420
ggcgaaaccc gacaggacta taaagatacc aggcgtttcc ccctggaagc tccctcgtgc 6480
gctctcctgt tccgaccctg ccgcttaccg gatacctgtc cgcctttctc ccttcgggaa 6540
gcgtggcgct ttctcatagc tcacgctgta ggtatctcag ttcggtgtag gtcgttcgct 6600
ccaagctggg ctgtgtgcac gaaccccccg ttcagcccga ccgctgcgcc ttatccggta 6660
actatcgtct tgagtccaac ccggtaagac acgacttatc gccactggca gcagccactg 6720
gtaacaggat tagcagagcg aggtatgtag gcggtgctac agagttcttg aagtggtggc 6780
ctaactacgg ctacactaga agaacagtat ttggtatctg cgctctgctg aagccagtta 6840

ccttcggaaa aagagttggt agctcttgat ccggcaaaca aaccaccgct ggtagcggtg 6900
gtttttttgt ttgcaagcag cagattacgc gcagaaaaaa aggatctcaa gaagatcctt 6960
tgatcttttc tacggggtct gacgctcagt ggaacgaaaa ctcacgttaa gggattttgg 7020
tcatgagatt atcaaaaagg atcttcacct agatcctttt aaattaaaaa tgaagtttta 7080
aatcaatcta aagtatatat gagtaaactt ggtctgacag ttaccaatgc ttaatcagtg 7140
aggcacctat ctcagcgatc tgtctatttc gttcatccat agttgcctga ctccccgtcg 7200
tgtagataac tacgatacgg gagggcttac catctggccc cagtgctgca atgataccgc 7260
gagacccacg ctcaccggct ccagatttat cagcaataaa ccagccagcc ggaagggccg 7320
agcgcagaag tggtcctgca actttatccg cctccatcca gtctattaat tgttgccggg 7380
aagctagagt aagtagttcg ccagttaata gtttgcgcaa cgttgttgcc attgctacag 7440
gcatcgtggt gtcacgctcg tcgtttggta tggcttcatt cagctccggt tcccaacgat 7500
caaggcgagt tacatgatcc cccatgttgt gcaaaaaagc ggttagctcc ttcggtcctc 7560
cgatcgttgt cagaagtaag ttggccgcag tgttatcact catggttatg gcagcactgc 7620
ataattctct tactgtcatg ccatccgtaa gatgcttttc tgtgactggt gagtactcaa 7680
ccaagtcatt ctgagaatag tgtatgcggc gaccgagttg ctcttgcccg gcgtcaatac 7740
gggataatac cgcgccacat agcagaactt taaaagtgct catcattgga aaacgttctt 7800
cggggcgaaa actctcaagg atcttaccgc tgttgagatc cagttcgatg taacccactc 7860
gtgcacccaa ctgatcttca gcatctttta ctttcaccag cgtttctggg tgagcaaaaa 7920
caggaaggca aaatgccgca aaaaagggaa taagggcgac acggaaatgt tgaatactca 7980
tactcttcct ttttcaatat tattgaagca tttatcaggg ttattgtctc atgagcggat 8040
acatatttga atgtatttag aaaaataaac aaataggggt tccgcgcaca tttccccgaa 8100
aagtgccacc tgacgtcgac ggatcgggag atcgatctcc cgatccccta gggtcgactc 8160
tcagtacaat ctgctctgat gccgcatagt taagccagta tctgctccct gcttgtgtgt 8220
tggaggtcgc tgagtagtgc gcgagcaaaa tttaagctac aacaaggcaa ggcttgaccg 8280
acaattgcat gaagaatctg cttagggtta ggcgttttgc gctgcttcgc gatgtacggg 8340
ccagatatac gcgttgacat tgattattga ctagttatta atagtaatca attacggggt 8400
cattagttca tagcccatat atggagttcc gcgttacata acttacggta aatggcccgc 8460
ctggctgacc gcccaacgac ccccgcccat tgacgtcaat aatgacgtat gttcccatag 8520
taacgccaat agggactttc cattgacgtc aatgggtgga gtatttacgg taaactgccc 8580
acttggcagt acatcaagtg tatc 8604

<210> 10
<211> 8532
<212> DNA
<213> Artificial Sequence

<400> 10

atatgccaag tacgccccct attgacgtca atgacggtaa atggcccgcc tggcattatg 60
cccagtacat gaccttatgg gactttccta cttggcagta catctacgta ttagtcatcg 120
ctattaccat ggtgatgcgg ttttggcagt acatcaatgg gcgtggatag cggtttgact 180
cacggggatt tccaagtctc caccccattg acgtcaatgg gagtttgttt tggcaccaaa 240
atcaacggga ctttccaaaa tgtcgtaaca actccgcccc attgacgcaa atgggcggta 300
ggcgtgtacg gtgggaggtc tatataagca gagctggttt agtgaaccgt cagatccgct 360
agagatccgc ggccgctaat acgactcact atagggagag ccgccaccat gagctcagag 420
actggcccag tggctgtgga ccccacattg agacggcgga tcgagcccca tgagtttgag 480
gtattcttcg atccgagaga gctccgcaag gagacctgcc tgctttacga aattaattgg 540
gggggccggc actccatttg gcgacataca tcacagaaca ctaacaagca cgtcgaagtc 600
aacttcatcg agaagttcac gacagaaaga tatttctgtc cgaacacaag gtgcagcatt 660
acctggtttc tcagctacag cccatgcggc gaatgtagta gggccatcac tgaattcctg 720
tcaaggtatc cccacgtcac tctgtttatt tacatcgcaa ggctgtacca ccacgctgac 780
cccgagaatc gacaaggcct gcgggatttg atctcttcag gtgtgactat ccaaattatg 840
actgagcagg agtcaggata ctgctggaga aactttgtga attatagccc gagtaatgaa 900
gcccactggc ctaggtatcc ccatctgtgg gtacgactgt acgttcttga actgtactgc 960
atcatactgg gcctgcctcc ttgtctcaac attctgagaa ggaagcagcc acagctgaca 1020
ttctttacca tcgctcttca gtcttgtcat taccagcgac tgcccccaca cattctctgg 1080
gccaccgggt tgaaaagcgg cagcgagact cccgggacct cagagtccgc cacacccgaa 1140

agtgataaaa agtattctat tggtttagcc atcggcacta attccgttgg atgggctgtc 1200
ataaccgatg aatacaaagt accttcaaag aaatttaagg tgttggggaa cacagaccgt 1260
cattcgatta aaaagaatct tatcggtgcc ctcctattcg atagtggcga aacggcagag 1320
gcgactcgcc tgaaacgaac cgctcggaga aggtatacac gtcgcaagaa ccgaatatgt 1380
tacttacaag aaatttttag caatgagatg gccaaagttg acgattcttt ctttcaccgt 1440
ttggaagagt ccttccttgt cgaagaggac aagaaacatg aacggcaccc catctttgga 1500
aacatagtag atgaggtggc atatcatgaa aagtacccaa cgatttatca cctcagaaaa 1560
aagctagttg actcaactga taaagcggac ctgaggttaa tctacttggc tcttgcccat 1620
atgataaagt tccgtgggca ctttctcatt gagggtgatc taaatccgga caactcggat 1680
gtcgacaaac tgttcatcca gttagtacaa acctataatc agttgtttga agagaaccct 1740
ataaatgcaa gtggcgtgga tgcgaaggct attcttagcg cccgcctctc taaatcccga 1800
cggctagaaa acctgatcgc acaattaccc ggagagaaga aaaatgggtt gttcggtaac 1860
cttatagcgc tctcactagg cctgacacca aattttaagt cgaacttcga cttagctgaa 1920
gatgccaaat tgcagcttag taaggacacg tacgatgacg atctcgacaa tctactggca 1980
caaattggag atcagtatgc ggacttattt ttggctgcca aaaaccttag cgatgcaatc 2040
ctcctatctg acatactgag agttaatact gagattacca aggcgccgtt atccgcttca 2100
atgatcaaaa ggtacgatga acatcaccaa gacttgacac ttctcaaggc cctagtccgt 2160
cagcaactgc ctgagaaata taaggaaata ttctttgatc agtcgaaaaa cgggtacgca 2220
ggttatattg acggcggagc gagtcaagag gaattctaca agtttatcaa acccatatta 2280
gagaagatgg atgggacgga agagttgctt gtaaaactca atcgcgaaga tctactgcga 2340
aagcagcgga ctttcgacaa cggtagcatt ccacatcaaa tccacttagg cgaattgcat 2400
gctatactta gaaggcagga ggatttttat ccgttcctca aagacaatcg tgaaaagatt 2460
gagaaaatcc taacctttcg cataccttac tatgtgggac ccctggcccg agggaactct 2520
cggttcgcat ggatgacaag aaagtccgaa gaaacgatta ctccatggaa ttttgaggaa 2580
gttgtcgata aaggtgcgtc agctcaatcg ttcatcgaga ggatgaccaa ctttgacaag 2640
aatttaccga acgaaaaagt attgcctaag cacagtttac tttacgagta tttcacagtg 2700
tacaatgaac tcacgaaagt taagtatgtc actgagggca tgcgtaaacc cgcctttcta 2760
agcggagaac agaagaaagc aatagtagat ctgttattca agaccaaccg caaagtgaca 2820
gttaagcaat tgaaagagga ctactttaag aaaattgaat gcttcgattc tgtcgagatc 2880
tccggggtag aagatcgatt taatgcgtca cttggtacgt atcatgacct cctaaagata 2940
attaaagata aggacttcct ggataacgaa gagaatgaag atatcttaga agatatagtg 3000
ttgactctta ccctctttga agatcgggaa atgattgagg aaagactaaa aacatacgct 3060
cacctgttcg acgataaggt tatgaaacag ttaaagaggc gtcgctatac gggctgggga 3120
cgattgtcgc ggaaacttat caacgggata agagacaagc aaagtggtaa aactattctc 3180
gattttctaa agagcgacgg cttcgccaat aggaacttta tgcagctgat ccatgatgac 3240
tctttaacct tcaaagagga tatacaaaag gcacaggttt ccggacaagg ggactcattg 3300
cacgaacata ttgcgaatct tgctggttcg ccagccatca aaaagggcat actccagaca 3360
gtcaaagtag tggatgagct agttaaggtc atgggacgtc acaaaccgga aaacattgta 3420
atcgagatgg cacgcgaaaa tcaaacgact cagaaggggc aaaaaaacag tcgagagcgg 3480
atgaagagaa tagaagaggg tattaaagaa ctgggcagcc agatcttaaa ggagcatcct 3540
gtggaaaata cccaattgca gaacgagaaa ctttacctct attacctaca aaatggaagg 3600
gacatgtatg ttgatcagga actggacata aaccgtttat ctgattacga cgtcgatcac 3660
attgtacccc aatccttttt gaaggacgat tcaatcgaca ataaagtgct tacacgctcg 3720
gataagaacc gagggaaaag tgacaatgtt ccaagcgagg aagtcgtaaa gaaaatgaag 3780
aactattggc ggcagctcct aaatgcgaaa ctgataacgc aaagaaagtt cgataactta 3840
actaaagctg agaggggtgg cttgtctgaa cttgacaagg ccggatttat taaacgtcag 3900
ctcgtggaaa cccgccaaat cacaaagcat gttgcacaga tactagattc ccgaatgaat 3960
acgaaatacg acgagaacga taagctgatt cgggaagtca aagtaatcac tttaaagtca 4020
aaattggtgt cggacttcag aaaggatttt caattctata aagttaggga gataaataac 4080
taccaccatg cgcacgacgc ttatcttaat gccgtcgtag ggaccgcact cattaagaaa 4140
tacccgaagc tagaaagtga gtttgtgtat ggtgattaca aagtttatga cgtccgtaag 4200
atgatcgcga aaagcgaaca ggagataggc aaggctacag ccaaatactt cttttattct 4260
aacattatga atttctttaa gacggaaatc actctggcaa acggagagat acgcaaacga 4320
cctttaattg aaaccaatgg ggagacaggt gaaatcgtat gggataaggg ccgggacttc 4380
gcgacggtga gaaaagtttt gtccatgccc caagtcaaca tagtaaagaa aactgaggtg 4440
cagaccggag ggttttcaaa ggaatcgatt cttccaaaaa ggaatagtga taagctcatc 4500
gctcgtaaaa aggactggga cccgaaaaag tacggtggct tcgatagccc tacagttgcc 4560
tattctgtcc tagtagtggc aaaagttgag aagggaaaat ccaagaaact gaagtcagtc 4620

aaagaattat tggggataac gattatggag cgctcgtctt ttgaaaagaa ccccatcgac 4680
ttccttgagg cgaaaggtta caaggaagta aaaaaggatc tcataattaa actaccaaag 4740
tatagtctgt ttgagttaga aaatggccga aaacggatgt tggctagcgc cggagagctt 4800
caaaagggga acgaactcgc actaccgtct aaatacgtga atttcctgta tttagcgtcc 4860
cattacgaga agttgaaagg ttcacctgaa gataacgaac agaagcaact ttttgttgag 4920
cagcacaaac attatctcga cgaaatcata gagcaaattt cggaattcag taagagagtc 4980
atcctagctg atgccaatct ggacaaagta ttaagcgcat acaacaagca cagggataaa 5040
cccatacgtg agcaggcgga aaatattatc catttgttta ctcttaccaa cctcggcgct 5100
ccagccgcat tcaagtattt tgacacaacg atagatcgca aacgatacac ttctaccaag 5160
gaggtgctag acgcgacact gattcaccaa tccatcacgg gattatatga aactcggata 5220
gatttgtcac agcttggggg tgactctggt ggttctacta atctgtcaga tattattgaa 5280
aaggagaccg gtaagcaact ggttatccag gaatccatcc tcatgctccc agaggaggtg 5340
gaagaagtca ttgggaacaa gccggaaagc gatatactcg tgcacaccgc ctacgacgag 5400
agcaccgacg agaatgtcat gcttctgact agcgacgccc ctgaatacaa gccttgggct 5460
ctggtcatac aggatagcaa cggtgagaac aagattaaga tgctctctgg tggttctccc 5520
aagaagaaga ggaaagtcta accggtcatc atcaccatca ccattgagtt taaacccgct 5580
gatcagcctc gactgtgcct tctagttgcc agccatctgt tgtttgcccc tcccccgtgc 5640
cttccttgac cctggaaggt gccactccca ctgtcctttc ctaataaaat gaggaaattg 5700
catcgcattg tctgagtagg tgtcattcta ttctgggggg tggggtgggg caggacagca 5760
agggggagga ttgggaagac aatagcaggc atgctgggga tgcggtgggc tctatggctt 5820
ctgaggcgga aagaaccagc tggggctcga taccgtcgac ctctagctag agcttggcgt 5880
aatcatggtc atagctgttt cctgtgtgaa attgttatcc gctcacaatt ccacacaaca 5940
tacgagccgg aagcataaag tgtaaagcct agggtgccta atgagtgagc taactcacat 6000
taattgcgtt gcgctcactg cccgctttcc agtcgggaaa cctgtcgtgc cagctgcatt 6060
aatgaatcgg ccaacgcgcg gggagaggcg gtttgcgtat tgggcgctct tccgcttcct 6120
cgctcactga ctcgctgcgc tcggtcgttc ggctgcggcg agcggtatca gctcactcaa 6180
aggcggtaat acggttatcc acagaatcag gggataacgc aggaaagaac atgtgagcaa 6240
aaggccagca aaaggccagg aaccgtaaaa aggccgcgtt gctggcgttt ttccataggc 6300
tccgcccccc tgacgagcat cacaaaaatc gacgctcaag tcagaggtgg cgaaacccga 6360
caggactata aagataccag gcgtttcccc ctggaagctc cctcgtgcgc tctcctgttc 6420
cgaccctgcc gcttaccgga tacctgtccg cctttctccc ttcgggaagc gtggcgcttt 6480
ctcatagctc acgctgtagg tatctcagtt cggtgtaggt cgttcgctcc aagctgggct 6540
gtgtgcacga accccccgtt cagcccgacc gctgcgcctt atccggtaac tatcgtcttg 6600
agtccaaccc ggtaagacac gacttatcgc cactggcagc agccactggt aacaggatta 6660
gcagagcgag gtatgtaggc ggtgctacag agttcttgaa gtggtggcct aactacggct 6720
acactagaag aacagtattt ggtatctgcg ctctgctgaa gccagttacc ttcggaaaaa 6780
gagttggtag ctcttgatcc ggcaaacaaa ccaccgctgg tagcggtggt ttttttgttt 6840
gcaagcagca gattacgcgc agaaaaaaag gatctcaaga agatcctttg atcttttcta 6900
cggggtctga cgctcagtgg aacgaaaact cacgttaagg gattttggtc atgagattat 6960
caaaaaggat cttcacctag atccttttaa attaaaaatg aagttttaaa tcaatctaaa 7020
gtatatatga gtaaacttgg tctgacagtt accaatgctt aatcagtgag gcacctatct 7080
cagcgatctg tctatttcgt tcatccatag ttgcctgact ccccgtcgtg tagataacta 7140
cgatacggga gggcttacca tctggcccca gtgctgcaat gataccgcga gacccacgct 7200
caccggctcc agatttatca gcaataaacc agccagccgg aagggccgag cgcagaagtg 7260
gtcctgcaac tttatccgcc tccatccagt ctattaattg ttgccgggaa gctagagtaa 7320
gtagttcgcc agttaatagt ttgcgcaacg ttgttgccat tgctacaggc atcgtggtgt 7380
cacgctcgtc gtttggtatg gcttcattca gctccggttc ccaacgatca aggcgagtta 7440
catgatcccc catgttgtgc aaaaaagcgg ttagctcctt cggtcctccg atcgttgtca 7500
gaagtaagtt ggccgcagtg ttatcactca tggttatggc agcactgcat aattctctta 7560
ctgtcatgcc atccgtaaga tgcttttctg tgactggtga gtactcaacc aagtcattct 7620
gagaatagtg tatgcggcga ccgagttgct cttgcccggc gtcaatacgg gataataccg 7680
cgccacatag cagaacttta aaagtgctca tcattggaaa acgttcttcg gggcgaaaac 7740
tctcaaggat cttaccgctg ttgagatcca gttcgatgta acccactcgt gcacccaact 7800
gatcttcagc atcttttact ttcaccagcg tttctgggtg agcaaaaaca ggaaggcaaa 7860
atgccgcaaa aaagggaata agggcgacac ggaaatgttg aatactcata ctcttccttt 7920
ttcaatatta ttgaagcatt tatcagggtt attgtctcat gagcggatac atatttgaat 7980
gtatttagaa aaataaacaa ataggggttc cgcgcacatt tccccgaaaa gtgccacctg 8040
acgtcgacgg atcgggagat cgatctcccg atcccctagg gtcgactctc agtacaatct 8100

gctctgatgc cgcatagtta agccagtatc tgctccctgc ttgtgtgttg gaggtcgctg 8160
agtagtgcgc gagcaaaatt taagctacaa caaggcaagg cttgaccgac aattgcatga 8220
agaatctgct tagggttagg cgttttgcgc tgcttcgcga tgtacgggcc agatatacgc 8280
gttgacattg attattgact agttattaat agtaatcaat tacggggtca ttagttcata 8340
gcccatatat ggagttccgc gttacataac ttacggtaaa tggcccgcct ggctgaccgc 8400
ccaacgaccc ccgcccattg acgtcaataa tgacgtatgt tcccatagta acgccaatag 8460
ggactttcca ttgacgtcaa tgggtggagt atttacggta aactgcccac ttggcagtac 8520
atcaagtgta tc 8532

<210> 11
<211> 2791
<212> DNA
<213> Artificial Sequence

<400> 11

tcgcgcgttt cggtgatgac ggtgaaaacc tctgacacat gcagctcccg gagacggtca 60
cagcttgtct gtaagcggat gccgggagca gacaagcccg tcagggcgcg tcagcgggtg 120
ttggcgggtg tcggggctgg cttaactatg cggcatcaga gcagattgta ctgagagtgc 180
accatatgcg gtgtgaaata ccgcacagat gcgtaaggag aaaataccgc atcaggcgcc 240
attcgccatt caggctgcgc aactgttggg aagggcgatc ggtgcgggcc tcttcgctat 300
tacgccagct ggcgaaaggg ggatgtgctg caaggcgatt aagttgggta acgccagggt 360
tttcccagtc acgacgttgt aaaacgacgg ccagtgaatt cgagctcggt acctcgcgaa 420
tgcatctaga tatcggatcc ctaatacgac tcactatagg cgccaatggt gttaacacat 480
gttttagagc tagaaatagc aagttaaaat aaggctagtc cgttatcaac ttgaaaaagt 540
ggcaccgagt cggtgctttt tttaaagggc ccgtcgactg cagaggcctg catgcaagct 600
tggcgtaatc atggtcatag ctgtttcctg tgtgaaattg ttatccgctc acaattccac 660
acaacatacg agccggaagc ataaagtgta aagcctgggg tgcctaatga gtgagctaac 720
tcacattaat tgcgttgcgc tcactgcccg ctttccagtc gggaaacctg tcgtgccagc 780
tgcattaatg aatcggccaa cgcgcgggga gaggcggttt gcgtattggg cgcggccgcc 840
gcttcctcgc tcactgactc gctgcgctcg gtcgttcggc tgcggcgagc ggtatcagct 900
cactcaaagg cggtaatacg gttatccaca gaatcagggg ataacgcagg aaagaacatg 960
tgagcaaaag gccagcaaaa ggccaggaac cgtaaaaagg ccgcgttgct ggcgtttttc 1020
cataggctcc gcccccctga cgagcatcac aaaaatcgac gctcaagtca gaggtggcga 1080
aacccgacag gactataaag ataccaggcg tttccccctg gaagctccct cgtgcgctct 1140
cctgttccga ccctgccgct taccggatac ctgtccgcct ttctcccttc gggaagcgtg 1200
gcgctttctc atagctcacg ctgtaggtat ctcagttcgg tgtaggtcgt tcgctccaag 1260
ctgggctgtg tgcacgaacc ccccgttcag cccgaccgct gcgccttatc cggtaactat 1320
cgtcttgagt ccaacccggt aagacacgac ttatcgccac tggcagcagc cactggtaac 1380
aggattagca gagcgaggta tgtaggcggt gctacagagt tcttgaagtg gtggcctaac 1440
tacggctaca ctagaagaac agtatttggt atctgcgctc tgctgaagcc agttaccttc 1500
ggaaaaagag ttggtagctc ttgatccggc aaacaaacca ccgctggtag cggtggtttt 1560
tttgtttgca agcagcagat tacgcgcaga aaaaaaggat ctcaagaaga tcctttgatc 1620
ttttctacgg ggtctgacgc tcagtggaac gaaaactcac gttaagggat tttggtcatg 1680
agattatcaa aaaggatctt cacctagatc cttttaaatt aaaaatgaag ttttaaatca 1740
atctaaagta tatatgagta aacttggtct gacagttaga aaaactcatc gagcatcaaa 1800
tgaaactgca atttattcat atcaggatta tcaataccat atttttgaaa aagccgtttc 1860
tgtaatgaag gagaaaactc accgaggcag ttccatagga tggcaagatc ctggtatcgg 1920
tctgcgattc cgactcgtcc aacatcaata caacctatta atttcccctc gtcaaaaata 1980
aggttatcaa gtgagaaatc accatgagtg acgactgaat ccggtgagaa tggcaaaagt 2040
ttatgcattt ctttccagac ttgttcaaca ggccagccat tacgctcgtc atcaaaatca 2100
ctcgcatcaa ccaaaccgtt attcattcgt gattgcgcct gagcgagacg aaatacgcga 2160
tcgctgttaa aaggacaatt acaaacagga atcgaatgca accggcgcag gaacactgcc 2220
agcgcatcaa caatattttc acctgaatca ggatattctt ctaatacctg gaatgctgtt 2280
ttcccaggga tcgcagtggt gagtaaccat gcatcatcag gagtacggat aaaatgcttg 2340
atggtcggaa gaggcataaa ttccgtcagc cagtttagtc tgaccatctc atctgtaaca 2400
tcattggcaa cgctaccttt gccatgtttc agaaacaact ctggcgcatc gggcttccca 2460
tacaatcgat agattgtcgc acctgattgc ccgacattat cgcgagccca tttataccca 2520

tataaatcag catccatgtt ggaatttaat cgcggcctag agcaagacgt ttcccgttga 2580
atatggctca tactcttcct ttttcaatat tattgaagca tttatcaggg ttattgtctc 2640
atgagcggat acatatttga atgtatttag aaaaataaac aaataggggt tccgcgcaca 2700
tttccccgaa aagtgccacc tgacgtctaa gaaaccatta ttatcatgac attaacctat 2760
aaaaataggc gtatcacgag gccctttcgt c 2791

<210> 12
<211> 4950
<212> DNA
<213> Artificial Sequence

<400> 12

ggtaccgatt agtgaacgga tctcgacggt atcgatcacg agactagcct cgagcggccg 60
cccccttcac cgagggccta tttcccatga ttccttcata tttgcatata cgatacaagg 120
ctgttagaga gataattgga attaatttga ctgtaaacac aaagatatta gtacaaaata 180
cgtgacgtag aaagtaataa tttcttgggt agtttgcagt tttaaaatta tgttttaaaa 240
tggactatca tatgcttacc gtaacttgaa agtatttcga tttcttggct ttatatatct 300
tgtggaaagg acgaaacacc gctacgtgtt aacaccattg ggttttagag ctagaaatag 360
caagttaaaa taaggctagt ccgttatcaa cttgaaaaag tggcaccgag tcggtgcttt 420
ttttaaagaa ttctcgacct cgagacaaat ggcagtattc atccacaatt ttaaaagaaa 480
aggggggatt ggggggtaca gtgcagggga aagaatagta gacataatag caacagacat 540
acaaactaaa gaattacaaa aacaaattac aaaaattcaa aattttcggg tttattacag 600
ggacagcaga gatccacttt ggccgcggct cgagggggtt ggggttgcgc cttttccaag 660
gcagccctgg gtttgcgcag ggacgcggct gctctgggcg tggttccggg aaacgcagcg 720
gcgccgaccc tgggactcgc acattcttca cgtccgttcg cagcgtcacc cggatcttcg 780
ccgctaccct tgtgggcccc ccggcgacgc ttcctgctcc gcccctaagt cgggaaggtt 840
ccttgcggtt cgcggcgtgc cggacgtgac aaacggaagc cgcacgtctc actagtaccc 900
tcgcagacgg acagcgccag ggagcaatgg cagcgcgccg accgcgatgg gctgtggcca 960
atagcggctg ctcagcaggg cgcgccgaga gcagcggccg ggaaggggcg gtgcgggagg 1020
cggggtgtgg ggcggtagtg tgggccctgt tcctgcccgc gcggtgttcc gcattctgca 1080
agcctccgga gcgcacgtcg gcagtcggct ccctcgttga ccgaatcacc gacctctctc 1140
cccaggggga tccaccggag cttaccatga ccgagtacaa gcccacggtg cgcctcgcca 1200
cccgcgacga cgtccccagg gccgtacgca ccctcgccgc cgcgttcgcc gactaccccg 1260
ccacgcgcca caccgtcgat ccggaccgcc acatcgagcg ggtcaccgag ctgcaagaac 1320
tcttcctcac gcgcgtcggg ctcgacatcg gcaaggtgtg ggtcgcggac gacggcgccg 1380
cggtggcggt ctggaccacg ccggagagcg tcgaagcggg ggcggtgttc gccgagatcg 1440
gcccgcgcat ggccgagttg agcggttccc ggctggccgc gcagcaacag atggaaggcc 1500
tcctggcgcc gcaccggccc aaggagcccg cgtggttcct ggccaccgtc ggcgtctcgc 1560
ccgaccacca gggcaagggt ctgggcagcg ccgtcgtgct ccccggagtg gaggcggccg 1620
agcgcgccgg ggtgcccgcc ttcctggaaa cctccgcgcc ccgcaacctc cccttctacg 1680
agcggctcgg cttcaccgtc accgccgacg tcgaggtgcc cgaaggaccg cgcacctggt 1740
gcatgacccg caagcccggt gcctgacgcc cgccccacga cccgcagcgc ccgaccgaaa 1800
ggagcgcacg accccatgca tcggtacctt taagaccaat gacttacaag gcagctgtag 1860
atcttagcca ctttctagag tcggggcggc cggccgcttc gagcagacat gataagatac 1920
attgatgagt ttggacaaac cacaactaga atgcagtgaa aaaaatgctt tatttgtgaa 1980
atttgtgatg ctattgcttt atttgtaacc attataagct gcaataaaca agttaacaac 2040
aacaattgca ttcattttat gtttcaggtt cagggggagg tgtgggaggt tttttaaagc 2100
aagtaaaacc tctacaaatg tggtaaaatc gataaggatc cgtcgaccga tgcccttgag 2160
agccttcaac ccagtcagct ccttccggtg ggcgcggggc atgactatcg tcgccgcact 2220
tatgactgtc ttctttatca tgcaactcgt aggacaggtg ccggcagcgc tcttccgctt 2280
cctcgctcac tgactcgctg cgctcggtcg ttcggctgcg gcgagcggta tcagctcact 2340
caaaggcggt aatacggtta tccacagaat caggggataa cgcaggaaag aacatgtgag 2400
caaaaggcca gcaaaaggcc aggaaccgta aaaaggccgc gttgctggcg tttttccata 2460
ggctccgccc ccctgacgag catcacaaaa atcgacgctc aagtcagagg tggcgaaacc 2520
cgacaggact ataaagatac caggcgtttc cccctggaag ctccctcgtg cgctctcctg 2580
ttccgaccct gccgcttacc ggatacctgt ccgcctttct cccttcggga agcgtggcgc 2640

tttctcaatg ctcacgctgt aggtatctca gttcggtgta ggtcgttcgc tccaagctgg 2700
gctgtgtgca cgaacccccc gttcagcccg accgctgcgc cttatccggt aactatcgtc 2760
ttgagtccaa cccggtaaga cacgacttat cgccactggc agcagccact ggtaacagga 2820
ttagcagagc gaggtatgta ggcggtgcta cagagttctt gaagtggtgg cctaactacg 2880
gctacactag aaggacagta tttggtatct gcgctctgct gaagccagtt accttcggaa 2940
aaagagttgg tagctcttga tccggcaaac aaaccaccgc tggtagcggt ggtttttttg 3000
tttgcaagca gcagattacg cgcagaaaaa aaggatctca agaagatcct ttgatctttt 3060
ctacggggtc tgacgctcag tggaacgaaa actcacgtta agggattttg gtcatgagat 3120
tatcaaaaag gatcttcacc tagatccttt taaattaaaa atgaagtttt aaatcaatct 3180
aaagtatata tgagtaaact tggtctgaca gttaccaatg cttaatcagt gaggcaccta 3240
tctcagcgat ctgtctattt cgttcatcca tagttgcctg actccccgtc gtgtagataa 3300
ctacgatacg ggagggctta ccatctggcc ccagtgctgc aatgataccg cgggacccac 3360
gctcaccggc tccagattta tcagcaataa accagccagc cggaagggcc gagcgcagaa 3420
gtggtcctgc aactttatcc gcctccatcc agtctattaa ttgttgccgg gaagctagag 3480
taagtagttc gccagttaat agtttgcgca acgttgttgc cattgctaca ggcatcgtgg 3540
tgtcacgctc gtcgtttggt atggcttcat tcagctccgg ttcccaacga tcaaggcgag 3600
ttacatgatc ccccatgttg tgcaaaaaag cggttagctc cttcggtcct ccgatcgttg 3660
tcagaagtaa gttggccgca gtgttatcac tcatggttat ggcagcactg cataattctc 3720
ttactgtcat gccatccgta agatgctttt ctgtgactgg tgagtactca accaagtcat 3780
tctgagaata gtgtatgcgg cgaccgagtt gctcttgccc ggcgtcaata cgggataata 3840
ccgcgccaca tagcagaact ttaaaagtgc tcatcattgg aaaacgttct tcggggcgaa 3900
aactctcaag gatcttaccg ctgttgagat ccagttcgat gtaacccact cgtgcaccca 3960
actgatcttc agcatctttt actttcacca gcgtttctgg gtgagcaaaa acaggaaggc 4020
aaaatgccgc aaaaaaggga ataagggcga cacggaaatg ttgaatactc atactcttcc 4080
tttttcaata ttattgaagc atttatcagg gttattgtct catgagcgga tacatatttg 4140
aatgtattta gaaaaataaa caaatagggg ttccgcgcac atttccccga aaagtgccac 4200
ctgacgcgcc ctgtagcggc gcattaagcg cggcgggtgt ggtggttacg cgcagcgtga 4260
ccgctacact tgccagcgcc ctagcgcccg ctcctttcgc tttcttccct tcctttctcg 4320
ccacgttcgc cggctttccc cgtcaagctc taaatcgggg gctcccttta gggttccgat 4380
ttagtgcttt acggcacctc gaccccaaaa aacttgatta gggtgatggt tcacgtagtg 4440
ggccatcgcc ctgatagacg gtttttcgcc ctttgacgtt ggagtccacg ttctttaata 4500
gtggactctt gttccaaact ggaacaacac tcaaccctat ctcggtctat tcttttgatt 4560
tataagggat tttgccgatt tcggcctatt ggttaaaaaa tgagctgatt taacaaaaat 4620
ttaacgcgaa ttttaacaaa atattaacgt ttacaatttc ccattcgcca ttcaggctgc 4680
gcaactgttg ggaagggcga tcggtgcggg cctcttcgct attacgccag cccaagctac 4740
catgataagt aagtaatatt aaggtacggg aggtacttgg agcggccgca ataaaatatc 4800
tttattttca ttacatctgt gtgttggttt tttgtgtgaa tcgatagtac taacatacgc 4860
tctccatcaa aacaaaacga aacaaaacaa actagcaaaa taggctgtcc ccagtgcaag 4920
tgcaggtgcc agaacatttc tctatcgata 4950

<210> 13
<211> 2791
<212> DNA
<213> Artificial Sequence

<400> 13

tcgcgcgttt cggtgatgac ggtgaaaacc tctgacacat gcagctcccg gagacggtca 60
cagcttgtct gtaagcggat gccgggagca gacaagcccg tcagggcgcg tcagcgggtg 120
ttggcgggtg tcggggctgg cttaactatg cggcatcaga gcagattgta ctgagagtgc 180
accatatgcg gtgtgaaata ccgcacagat gcgtaaggag aaaataccgc atcaggcgcc 240
attcgccatt caggctgcgc aactgttggg aagggcgatc ggtgcgggcc tcttcgctat 300
tacgccagct ggcgaaaggg ggatgtgctg caaggcgatt aagttgggta acgccagggt 360
tttcccagtc acgacgttgt aaaacgacgg ccagtgaatt cgagctcggt acctcgcgaa 420
tgcatctaga tatcggatcc ctaatacgac tcactatagg ctacgtgtta acaccattgg 480
gttttagagc tagaaatagc aagttaaaat aaggctagtc cgttatcaac ttgaaaaagt 540
ggcaccgagt cggtgctttt tttaaagggc ccgtcgactg cagaggcctg catgcaagct 600
tggcgtaatc atggtcatag ctgtttcctg tgtgaaattg ttatccgctc acaattccac 660

acaacatacg agccggaagc ataaagtgta aagcctgggg tgcctaatga gtgagctaac 720
tcacattaat tgcgttgcgc tcactgcccg ctttccagtc gggaaacctg tcgtgccagc 780
tgcattaatg aatcggccaa cgcgcgggga gaggcggttt gcgtattggg cgcggccgcc 840
gcttcctcgc tcactgactc gctgcgctcg gtcgttcggc tgcggcgagc ggtatcagct 900
cactcaaagg cggtaatacg gttatccaca gaatcagggg ataacgcagg aaagaacatg 960
tgagcaaaag gccagcaaaa ggccaggaac cgtaaaaagg ccgcgttgct ggcgtttttc 1020
cataggctcc gcccccctga cgagcatcac aaaaatcgac gctcaagtca gaggtggcga 1080
aacccgacag gactataaag ataccaggcg tttccccctg gaagctccct cgtgcgctct 1140
cctgttccga ccctgccgct taccggatac ctgtccgcct ttctcccttc gggaagcgtg 1200
gcgctttctc atagctcacg ctgtaggtat ctcagttcgg tgtaggtcgt tcgctccaag 1260
ctgggctgtg tgcacgaacc ccccgttcag cccgaccgct gcgccttatc cggtaactat 1320
cgtcttgagt ccaacccggt aagacacgac ttatcgccac tggcagcagc cactggtaac 1380
aggattagca gagcgaggta tgtaggcggt gctacagagt tcttgaagtg gtggcctaac 1440
tacggctaca ctagaagaac agtatttggt atctgcgctc tgctgaagcc agttaccttc 1500
ggaaaaagag ttggtagctc ttgatccggc aaacaaacca ccgctggtag cggtggtttt 1560
tttgtttgca agcagcagat tacgcgcaga aaaaaaggat ctcaagaaga tcctttgatc 1620
ttttctacgg ggtctgacgc tcagtggaac gaaaactcac gttaagggat tttggtcatg 1680
agattatcaa aaaggatctt cacctagatc cttttaaatt aaaaatgaag ttttaaatca 1740
atctaaagta tatatgagta aacttggtct gacagttaga aaaactcatc gagcatcaaa 1800
tgaaactgca atttattcat atcaggatta tcaataccat atttttgaaa aagccgtttc 1860
tgtaatgaag gagaaaactc accgaggcag ttccatagga tggcaagatc ctggtatcgg 1920
tctgcgattc cgactcgtcc aacatcaata caacctatta atttcccctc gtcaaaaata 1980
aggttatcaa gtgagaaatc accatgagtg acgactgaat ccggtgagaa tggcaaaagt 2040
ttatgcattt ctttccagac ttgttcaaca ggccagccat tacgctcgtc atcaaaatca 2100
ctcgcatcaa ccaaaccgtt attcattcgt gattgcgcct gagcgagacg aaatacgcga 2160
tcgctgttaa aaggacaatt acaaacagga atcgaatgca accggcgcag gaacactgcc 2220
agcgcatcaa caatattttc acctgaatca ggatattctt ctaatacctg gaatgctgtt 2280
ttcccaggga tcgcagtggt gagtaaccat gcatcatcag gagtacggat aaaatgcttg 2340
atggtcggaa gaggcataaa ttccgtcagc cagtttagtc tgaccatctc atctgtaaca 2400
tcattggcaa cgctaccttt gccatgtttc agaaacaact ctggcgcatc gggcttccca 2460
tacaatcgat agattgtcgc acctgattgc ccgacattat cgcgagccca tttataccca 2520
tataaatcag catccatgtt ggaatttaat cgcggcctag agcaagacgt ttcccgttga 2580
atatggctca tactcttcct ttttcaatat tattgaagca tttatcaggg ttattgtctc 2640
atgagcggat acatatttga atgtatttag aaaaataaac aaataggggt tccgcgcaca 2700
tttccccgaa aagtgccacc tgacgtctaa gaaaccatta ttatcatgac attaacctat 2760
aaaaataggc gtatcacgag gccctttcgt c 2791

<210> 14
<211> 24
<212> DNA
<213> Artificial Sequence

<400> 14
taggcgccaa tggtgttaac acat 24

<210> 15
<211> 24
<212> DNA
<213> Artificial Sequence

<400> 15
aaacatgtgt taacaccatt ggcg 24

<210> 16
<211> 24
<212> DNA
<213> Artificial Sequence

<400>  16
accgctacgt gttaacacca ttgg  24

<210>  17
<211>  24
<212>  DNA
<213>  Artificial Sequence

<400>  17
aaacccaatg gtgttaacac gtag  24

<210>  18
<211>  24
<212>  DNA
<213>  Artificial Sequence

<400>  18
taggctacgt gttaacacca ttgg  24

<210>  19
<211>  24
<212>  DNA
<213>  Artificial Sequence

<400>  19
aaacccaatg gtgttaacac gtag  24

<210>  20
<211>  20
<212>  DNA
<213>  Artificial Sequence

<400>  20
tttcccatga ttccttcata  20

<210>  21
<211>  24
<212>  DNA
<213>  Artificial Sequence

<400>  21
cgccagggtt ttcccagtca cgac  24

<210>  22
<211>  24
<212>  DNA
<213>  Artificial Sequence

<400>  22
tctcgcgcgt ttcggtgatg acgg  24

<210>  23
<211>  31
<212>  DNA
<213>  Artificial Sequence

<400>  23

aaaaaaagca ccgactcggt gccacttttt c  31

<210>  24
<211>  20
<212>  DNA
<213>  Artificial Sequence

<400>  24
actcaccaat gcaggacgta  20

<210>  25
<211>  20
<212>  DNA
<213>  Artificial Sequence

<400>  25
agctgcttca tagggtcagc  20

<210>  26
<211>  19
<212>  DNA
<213>  Artificial Sequence

<400>  26
gctgaagtct ccacccacc  19

<210>  27
<211>  19
<212>  DNA
<213>  Artificial Sequence

<400>  27

tgtctctcct tgccttttg  19

<210>  28
<211>  20
<212>  DNA
<213>  Artificial Sequence

<400>  28
   tcaagggaca ggagtaggca  20

<210>  29
<211>  20
<212>  DNA
<213>  Artificial Sequence

<400>  29
ttggggcagg aggttttgtt  20

<210>  30
<211>  19
<212>  DNA
<213>  Artificial Sequence

<400>  30
atcttaatca gggccttga  19

<210>  31
<211>  19
<212>  DNA
<213>  Artificial Sequence

<400>  31
gccttcattc catcaactg  19

<210>  32
<211>  20
<212>  DNA
<213>  Artificial Sequence

<400>  32
caggttcgtg tcgcagtagc  20

<210>  33
<211>  19
<212>  DNA
<213>  Artificial Sequence

<400>  33
ctgtgttgcc agcacgaaa  19

<210>  34
<211>  20
<212>  DNA
<213>  Artificial Sequence

<400>  34
tggtagtggt tggtgacact  20

<210>  35
<211>  20
<212>  DNA
<213>  Artificial Sequence

<400>  35
cgttacattg ggaagcggaa  20

<210>  36
<211>  20
<212>  DNA
<213>  Artificial Sequence

<400>  36
ggattcaaca tagattggaa  20

<210>  37
<211>  18
<212>  DNA
<213>  Artificial Sequence

<400>  37
cccgtttaca cattgcta  18

<210>  38

<211>  20
<212>  DNA
<213>  Artificial Sequence

<400>  38
ttctagtagg tgaaaaaggg  20

<210>  39
<211>  19
<212>  DNA
<213>  Artificial Sequence

<400>  39
ttggacacca catagacag  19

<210>  40
<211>  22
<212>  DNA
<213>  Artificial Sequence

<400>  40
tattattgct aaaccgaaac ca  22

<210>  41
<211>  18
<212>  DNA
<213>  Artificial Sequence

<400>  41
agcccctcac ccactcat  18

<210>  42
<211>  20
<212>  DNA
<213>  Artificial Sequence

<400>  42
agaggcttgc gaaggacatc  20

<210>  43
<211>  20
<212>  DNA
<213>  Artificial Sequence

<400>  43
atttggtcta gggcagaggc  20

<210>  44
<211>  20
<212>  DNA
<213>  Artificial Sequence

<400>  44
attattcaca agttatggta  20

<210>  45
<211>  18
<212>  DNA

<213>  Artificial Sequence

<400>  45
taaccctctt ctttgtaa  18

<210>  46
<211>  23
<212>  DNA
<213>  Artificial Sequence

<400>  46
aagggactgt ttttgtcctg tca  23

<210>  47
<211>  23
<212>  DNA
<213>  Artificial Sequence

<400>  47
gtgaaaccac catgacatga agt  23

<210>  48
<211>  20
<212>  DNA
<213>  Artificial Sequence

<400>  48
gtcatacttg gccagggtcc  20

<210>  49
<211>  20
<212>  DNA
<213>  Artificial Sequence

<400>  49
cccacgtgag ctggctaaaa  20

<210>  50
<211>  20
<212>  DNA
<213>  Artificial Sequence

<400>  50
tgatcagcat gtggagcctg  20

<210>  51
<211>  20
<212>  DNA
<213>  Artificial Sequence

<400>  51
gaagtcagcc aggagccatt  20

<210>  52
<211>  18
<212>  DNA
<213>  Artificial Sequence

<400>  52
gagttaggag tgggaagg  18

<210>  53
<211>  21
<212>  DNA
<213>  Artificial Sequence

<400>  53
acaaaggaca gtaatgaaga g  21

<210>  54
<211>  20
<212>  DNA
<213>  Artificial Sequence

<400>  54
tttgcctcct tgattccccc  20

<210>  55
<211>  20
<212>  DNA
<213>  Artificial Sequence

<400>  55
gtggatggtg tggaggtgag  20

<210>  56
<211>  22
<212>  DNA
<213>  Artificial Sequence

<400>  56
cgataaaggg atcagtcact aa  22

<210>  57
<211>  19
<212>  DNA
<213>  Artificial Sequence

<400>  57
gctccaggtc cacaaacac  19

## Description

### BACKGROUND

### Technical Field

The present invention relates to the field of gene repair, and more particularly to a method for repairing a Marfan syndrome-associated *FBN1^{T7498C}* mutation by using base editing.

### Related Art

To date, nearly 10,000 genetic diseases have been identified in medicine, placing a huge burden on families and society, yet only about 6% of genetic diseases are currently treatable (Austin and Dawkins, 2017). Diagnosing and treating genetic diseases has become an important research topic in medicine. The development of high-throughput sequencing technology has made the diagnosis of genetic diseases easier. Although pre-implantation genetic diagnosis (PGD) can block the occurrence of some genetic diseases, it is necessary to develop more effective genetic treatment means for some genetic diseases such as homozygous mutations (Dunbar et al., 2018).

The gene editing technology, especially CRISPR/Cas9, has been widely applied to gene manipulation and can be applied to accurately repair disease-causing mutations (Komor et al., 2017). At the same time, the technology has also shown great advantages in genetic editing of human embryos, suggesting its clinical value in the treatment of human genetic diseases (Kang et al., 2016). However, due to the limitation of the ethics and efficiency of treatment and the existence of off-target, there is still much room for improvement in the application of gene editing in human embryos (Ruzo and Brivanlou, 2017). CRISPR/Cas9-mediated gene editing is to allow an sgRNA (single guided RNA) to guide the Cas9 protein through target sequence complementation to position and cleave a double-stranded DNA, resulting in double-strand DNA breaks (DSB); in the absence of a template, non-homologous end joining (NHEJ) repair occurs to cause a frameshift mutation, resulting in gene knockout; in the presence of the template, homology-directed repair (HDR) is used to achieve gene knockin; the efficiency of HDR is low (integration rarely occurs), and a non-homologous end joining mechanism is easy to generate random insertions and deletions (indel), so that new bases may be introduced randomly near a breakpoint, thereby causing inaccurate gene editing (Hsu et al., 2014). In addition, CRISPR/Cas9-mediated gene editing always has some off-target effects [Gorski *et al.,* 2017].

A recently developed base editor based on a nicking enzyme nCas9 is added with rat cytosine deaminase APOBEC1. By means of the base editor, C on a target site can be converted to uracil without cleaving a DNA double strand (Komor et al., 2016). Thereafter, by DNA replication or repair, uracil is converted to thymine (T), thereby achieving a conversion from C to T, and similarly, a single base G can also be converted to A therethrough. Since DSB is caused without cleaving DNA, the formed indel is less than 1%, and the gene editing achieved is more accurate. The base editor has been successfully applied to in vivo base editing to achieve CT mutations in mice. We also use BE3 to achieve efficient editing of target sites in human discarded embryos.

The Marfan syndrome is an autosomal dominant genetic disease, accounting for 0.2‰ of the world's population. It mainly causes abnormal development of human connective tissues. At present, the sudden death of many well-known athletes is related to the Marfan syndrome (Arbustini et al., 2005). Although some patients can be treated surgically, there is still a risk of disease in their offspring, and the genetic treatment of mutations causing the disease will be the fundamental method. The mutation of the *FBN1* gene has been previously shown to be the main cause of the Marfan syndrome. Therefore, whether an effective and safe treatment method can be found is the direction we pursue. The present invention aims to find a method for more efficiently and safely repairing Marfan syndrome-associated mutations and repairing the mutations at the human embryo stage, thereby reducing the incidence and the huge social burden of the disease.

### References

Arbustini, E., Grasso, M., Ansaldi, S., Malattia, C., Pilotto, A., Porcu, E., Disabella, E., Marziliano, N., Pisani, A., Lanzarini, L., et al. (2005). Identification of sixty-two novel and twelve known FBN1 mutations in eighty-one unrelated probands with Marfan syndrome and other fibrillinopathies. Human mutation 26, 494.
Austin, C.P., and Dawkins, H.J.S. (2017). Medical research: Next decade's goals for rare diseases. Nature 548, 158.
Dunbar, C.E., High, K.A., Joung, J.K., Kohn, D.B., Ozawa, K., and Sadelain, M. (2018). Gene therapy comes of age. Science 359.
Hsu, P.D., Lander, E.S., and Zhang, F. (2014). Development and applications of CRISPR-Cas9 for genome engineering. Cell 157, 1262-1278.
Kang, X., He, W., Huang, Y., Yu, Q., Chen, Y., Gao, X., Sun, X., and Fan, Y. (2016). Introducing precise genetic modifications into human 3PN embryos by CRISPR/Cas-mediated genome editing. Journal of assisted reproduction and genetics 33, 581-588.
Komor, A.C., Badran, A.H., and Liu, D.R. (2017). CRISPR-Based Technologies for the Manipulation of Eukaryotic Genomes. Cell 168, 20-36.
Komor, A.C., Kim, Y.B., Packer, M.S., Zuris, J.A., and Liu, D.R. (2016). proceduremable editing of a target base in genomic DNA without double-stranded DNA cleavage. Nature 533, 420-424.
Ruzo, A., and Brivanlou, A.H. (2017). At Last: Gene Editing in Human Embryos to Understand Human Development. Cell Stem Cell 21, 564-565.

### SUMMARY

The object of the present invention is to provide a kit and a method for efficiently repairing an *FBN1^{T7498C}* mutation.

To achieve the above object, the present invention provides a kit for efficiently repairing an *FBN1^{T7498C}* mutation, which is characterized by comprising a base editor and a repair re-sgRNA directed to an *FBN1*^{*T*7498C} site.

Preferably, the base editor is one of BE3, YE1-BE3, YE2-BE3 or YEE-BE3.

Preferably, the sequence of the repair re-sgRNA directed to the *FBN1^{T7498C}* site is SEQ ID NO.3.

The present invention also provides a combination for making a mutation and repairing a mutation, which is characterized by comprising at least one of a mutated mt-sgRNA designed according to the *FBN1^{T7498C}* site and a corresponding mutated ssODN, a repair re-sgRNA directed to the *FBNI^{T7498C}* site, and a base editor.

The present invention also provides a method for repairing a mutation by using base editing, which is characterized by comprising: in an *FBN1^{T7498C}-containing* mutated cell, using a repair re-sgRNA directed to the *FBN1^{T7498C}* site to guide a base editor to a mutation site to perform base editing repair and collect transfected cells.

Preferably, the *FBN1^{T7498C}*-containing mutated cell is an HEK293T cell or embryonic cell.

Preferably, a method for constructing the *FBN1^{T7498C}*-containing mutated cell comprises: designing a mutated mt-sgRNA and a corresponding mutated ssODN according to the *FBN1^{T7498C}* site; constructing an expression vector of mt-sgRNA, and allowing Cas9 protein and transcribed mt-sgRNA in vitro to form an RNP complex to be electrotransfected into HEK293T cells with ssODN, and performing flow sorting on single cells and identifying an *FBN1^{T7498C}-*containing mutated cell line.

Preferably, the repair re-sgRNA directed to the *FBN1^{T7498C}* site is obtained by designing the *FBN1^{T7498C}* site and constructing a U6 started and/or T7 started expression vector.

Preferably, the method for repairing the Marfan disease mutation by base editing also comprises: detecting the repair efficiency by Sanger sequencing; injecting mRNA and re-sgRNA of the base editor into the human embryonic cell containing the *FBN1^{T7498C}* mutation, detecting the repair efficiency in the embryos, and detecting the on-target and off-target efficiency by high-throughput sequencing.

Preferably, the sequence of the mt-sgRNA is SEQ ID NO. 1, the sequence of ssODN is SEQ ID NO. 2, and the sequence of re-sgRNA is SEQ ID NO.3.

Preferably, a method for obtaining the embryos is to inject a sperm containing the *FBN1^{T7498C}* site mutation into a normal oocyte by ICSI, or to inject a normal sperm into an egg containing the *FBN1^{T7498C}* mutation site by ICSI, or to inject the sperm containing the *FBN1^{T7498C}* site mutation into an egg containing the *FBN1^{T7498C}* mutation site to obtain a heterozygous or homozygous mutated embryo containing this site.

The present invention also provides a method for repairing a Marfan disease mutation by base editing, comprising: designing a mutated mt-sgRNA according to the *FBN1^{T7498C}* site and a corresponding mutated ssODN; constructing a T7 started expression vector of mt-sgRNA, allowing a Cas9 protein and the transcribed mt-sgRNA in vitro to form an RNP to be bound to ssODN and electrotransfect 293T cells, and performing flow sorting on single cells and identifying an *FBN1^{T7498C}*-containing homozygous mutated cell line; designing a repair re-sgRNA according to the *FBN1^{T7498C}* site, and respectively constructing U6 started and T7 started expression vectors; in the made homozygous mutated cell line, using U6 started re-sgRNA to guide the base editor to the mutation site, collecting the transfected cells after 3 days, and detecting the repair efficiency by Sanger sequencing; injecting mRNA and re-sgRNA of BE3 into human embryonic cells containing the site mutation, detecting the repair efficiency in the embryos, and detecting the on-target and off-target efficiency by high-throughput sequencing.

The present invention proves the high efficiency and safety of the method at two different levels of cells and embryos by using the base editing technology.

The mutation of *FBN1* is the main cause of the Marfan syndrome. Its incidence rate is about 0.2‰, and the genetically complete repair of this mutation will be the most effective measure to treat the disease. The base editor provides a method for precisely changing DNA, namely, a method for converting C to T.

In the present invention, the mutated human embryos are repaired by using the base editor as a novel base editing tool. The safety and efficacy of the method will first be verified at two levels of cells and embryos. In terms of cells, the inventors will use a homologous recombination method based on CRISPR/Cas9 and ssODN to make a cell line containing the *FBN1^{T7498C}* mutation, and then use the base editor to be bound to a suitable sgRNA to repair the mutation at this site. After verifying the safety and efficacy of the system, the transcribed mRNA and sgRNA are injected into the human embryos containing the *FBN1^{T7498C}* mutation, the embryos are collected after three days, and the repair efficiency and the off-target situation are detected by a deep sequencing method.

In the present invention, the base editing technology is used to repair the *FBN1^{T7498C}* mutation through the precise CT single base mutation, thereby providing an efficient and safe method for treating the Marfan syndrome caused by such mutation.

### BRIEF DESCRIPTION OF THE DRAWINGS

Figure 1 shows the confirmation of mutation sites in samples from patients with a Marfan syndrome. (A) is sampled from blood, (B) is sampled from semen.
Figure 2 shows the making of mutated cell lines in 293T cells by means of Cas9/sgRNA bound to ssODN. (A) is a mode diagram of making a mutation and repairing a mutation in cells. (B) is design of mutation type sgRNA and ssODN for the *FBNI* gene to make corresponding mutations. (C) is T7EN1 enzyme digestion identification on transfection efficiency after transfecting cells. (D) is flow sorting of twenty-two single cell clones, and sanger sequencing confirmation of mutation types of cells. (E) is a sanger sequencing peak map of homozygous mutations.
Figure 3 shows off-target detection sorting of the mutated sgRNA. (A) is the analysis of potential off-target sites of the mutated sgRNA by software, and detection of corresponding off-target sites and detection of off-target by T7EN1 for the homozygous mutated cell lines. (B) is sanger sequencing detection on mutations.
Figure 4 shows the repair of mutated cell lines by using base editing. (A) is design of a corresponding repair sgRNA at the mutation site and repair by using base editing. (B) is sequencing detection on the mutation situation after cell transfection. (C) is TA clone identification of repair type of Figure B. (D) is a sanger sequencing peak map after complete repair.
Figure 5 shows repair of mutated cell lines by using YE1-BE3, YEE-BE3 and BE3.
Figure 6 shows off-target detection of repair sgRNA. (A) is enzyme digestion detection of off-target by using T7EN1. (B) is confirmation of off-target situation by using sanger sequencing.
Figure 7 shows repair of human mutated embryos. (A) is a schematic diagram of human embryo repair by using base editing. (B) shows an embryonic state after the embryos containing the mutations are injected with the relevant RNA. (C) is the genotype of representative embryos injected with the repair sgRNA. (D) is the genotype of representative embryos injected with a random sgRNA. (E) is genotypic analysis of repaired embryos and control embryos by high-throughput sequencing.
Figure 8 shows genotypes detected of the repaired embryos and control embryos used. (A) is the genotype of repaired embryos. (B) is the genotype of control embryos.
Figure 9 shows detection of off-target of the repair sgRNA by using high-throughput sequencing in the repaired embryos.

### DETAILED DESCRIPTION

The examples of the present invention will be clearly and completely described with reference to embodiments. It is apparent that the described embodiments are only intended to illustrate a part of the embodiments of the present invention and are not to be construed as limiting the scope of the present invention. If no specific conditions are specified in the embodiments, the embodiments are carried out according to conventional conditions or the conditions recommended by manufacturers. If reagents or instruments used are not labeled with the specific manufacturers, they are considered to be conventional products that can be purchased commercially.

The above is only the preferred embodiments of the present invention, and is not intended to limit the present invention. Any modifications, equivalent substitutions, improvements, etc. within the spirit and scope of the present invention should be included in the scope of protection of the present invention.

First, different versions of BE3, namely YE1-BE3, YE2-BE3, YEE-BE3, and BE3, were constructed. The original version BE3 was purchased from Addgene (73021).

### 1. Synthesis of different types of APOBEC1

The above four kinds of BE3 differ only in the coding frame of rAPOBEC1, and sequences of YE1-rAPOBEC1 (SEQ ID NO. 4), YE2-rAPOBEC1 (SEQ ID NO. 5), and YEE-rAPOBEC1 (SEQ ID NO. 6) were synthesized by Sangon Biotech (http://www.sangon.com/). The two ends of the synthesized fragments were respectively ligated with enzyme digestion sites of NotI and SmaI. The synthesized fragments were cloned onto a normally used pmd19t vector (TAKARA: 6013).

### 2. Enzyme digestion and purification of vectors

BE3 and the above three synthesized vectors were enzyme-digested with NotI (NEB:
R0189L) and SmaI (NEB: R0141L). The system was as follows: 6uL of Buffer (NEB: R0189L), 2ug of plasmid, 1µL of NotI, 1µL of SmaI and ddH₂O complemented to 60µL. After samples were mixed, the enzyme digestion was carried out overnight at a temperature of 37°C.

The enzyme-digested product was recovered by 1% of agarose gel (Axygen: AP-GX-250G). BE3 recovered the backbone vector of large fragments, and the synthesized vector recovered small fragment vector of APOBEC1. The recovery was carried out according to the instructions for use of a kit (Axygen: AP-PCR-250G). The concentrations of the recovered fragments were detected by Nanodrop 2000.

### 3. Ligation, transformation and plasmid extraction of vectors

The recovered backbone vector and the APOBEC1 fragment were ligated by the following system: 1 µL of T4 ligation buffer (NEB: M0202L), 20 ng of backbone vector, 50 ng of APOBEC1 fragment, 0.5 µL of T4 ligase (NEB: M0202L), and ddH₂O complemented to 10 µL. Ligation was carried out overnight at a temperature of 16°C. The transformation step comprises: taking 20 µL of competent cells (TransGen: CD201) and thawing on ice, mixing 2 µL of a ligation product with the competent cells, placing the mixture on ice for 20 minutes, performing heat-shocking at a temperature of 42°C for 60 seconds, placing the mixture on ice for 2 minutes, adding 400 µL of a resuscitation LB medium (MDBio: L001-1 kg) and shaking on a shaker for 30 minutes, taking 70 µL of an ampicillin-coated plate (50 µg/ml), and culturing in a 37°C incubator for 14 hours.

Monoclonal bacteria were selected and cultured in 4 ml of a liquid LB medium on a large scale, and the plasmids (Axygene: AP-MN-P-250G) were extracted after 14 hours. The step comprises: centrifuging a bacterial solution at a speed of 4,000 rpm for 10 minutes, and pouring a supernatant medium out; adding 350 µL of a buffer S1, blowing off the thalli, and transferring to a 2 ml centrifuge tube; adding 250 µL of a buffer S2 and putting upside down for 8 times; adding 250 µL of a buffer S3, putting upside down and mixing evenly for 6 times to produce a precipitate; centrifuging at a speed of 12,000 rpm for 10 minutes, taking a supernatant and performing column chromatography; centrifuging for 1 minute, pouring a waste solution out, adding 500 µL of W1, centrifuging for one minute, and pouring a waste solution out; adding 750 µL of W2, centrifuging, and pouring a supernatant out; adding 500 µL of W2, centrifuging, and pouring a supernatant out; idling for 1 minute; adding 50 µL of an eluate, keeping stand for 2 minutes, and centrifuging. The obtained plasmids were subjected to concentration detection, 10 µL of plasmids were taken for sequencing, and positive plasmids were stored at a temperature of -20°C.

Finally, four types of BE3 were constructed as follows:
(1) YE1-BE3, SEQ ID NO. 7;
(2) YE2-BE3, SEQ ID NO. 8;
(3) YEE-BE3, SEQ ID NO. 9;
(4) BE3, SEQ ID NO. 10.

In the case of performing the mutation site repair below, any one of the above BE3 may be used, preferably (1) or (4).

### Embodiment 1

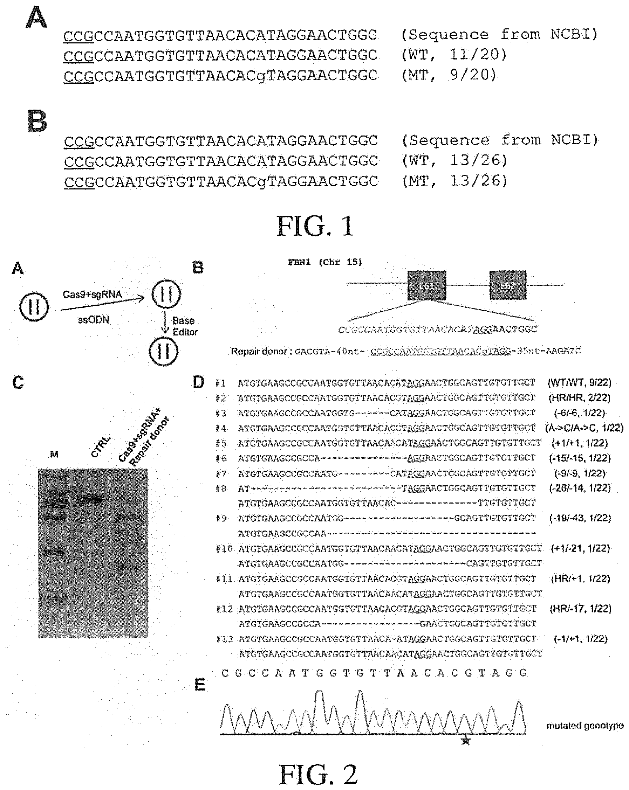

In this embodiment, a mutation *FBN1^{T7498C}* mutated cell line was made by using Cas9/sgRNA bound to ssODN on a cell line, and the mutated cell line was repaired by using a base editor (Figure 2).

### 1.1 Plasmid construction

Near a mutation site, a mutated mt-sgRNA (SEQ ID NO. 1) was designed and oligos were synthesized. The upstream sequence was 5'-taggCGCCAATGGTGTTAACACAT-3' (SEQ ID NO. (14)), the downstream sequence was 5'-aaac ATGTGTTAACACCATTGGCG-3' (SEQ ID NO. (15)), and the upstream and downstream sequences were annealed by a procedure (95°C, 5 min; 95°C to 85°C at - 2°C/s; 85°C to 25°C at - 0.1°C/s; hold at 4°C) and ligated to a PUC57-T7sgRNA vector (addgene: 51132) linearized with BsaI (NEB: R0539L). The linearization system was as follows: 2 µg of PUC57-T7sgRNA, 6 µL of buffer (NEB: R0539L), 2 µL of BsaI, and ddH₂O complemented to 60 µL. The enzyme digestion was carried out overnight at a temperature of 37°C. A homologous template ssODN (SEQ ID NO. 2) used was synthesized by means of PAGE purification by Sangon Biotech Company (http://www.sangon.com/). At the same time, near the mutation site, according to the characteristics of base editing, a repair re-sgRNA (SEQ ID NO. 3) was designed, and oligos were synthesized, the upstream sequence was 5'-accgCTACGTGTTAACACCATTGG-3' (SEQ ID NO. 16), and the downstream sequence was 5'-aaacCCAATGGTGTTAACACGTAG-3' (SEQ ID NO. 17). The upstream and downstream sequences were annealed by a procedure (95°C, 5 min; 95°C - 85°C at - 2°C/s; 85°C - 25°C at - 0.1°C/s; hold at 4°C), and ligated to a PGL3-U6sgRNA vector linearized with BsaI (NEB: R0539L). At the same time, an upstream primer: 5'-taggCTACGTGTTAACACCATTGG-3" (SEQ ID NO. 18) and a downstream primer: 5'-aaacCCAATGGTGTTAACACGTAG-3' (SEQ ID NO. 19) were synthesized and ligated to a linearized PUC57-T7sgRNA vector by annealing. The annealing procedure, the linearization system and procedure were as above. The ligation system was as follows: 1 µL of T4 ligation buffer (NEB: M0202L), 20 ng of linearized vector, 5 µL of annealed oligo fragment (10 µM), 0.5 µL of T4 ligase (NEB: M0202L), and ddH₂O complemented to 10 µL. Ligation was carried out overnight at a temperature of 16°C. The ligated vector was subjected to transformation, bacterium selection and identification, the U6 vector as the identification primer was the upstream sequence: 5'-TTTCCCATGATTCCTTCATA-3' (SEQ ID NO. 20), and the downstream sequence was the downstream sequence of the corresponding oligo. The T7 vector was the upstream sequence: 5'-CGCCAGGGTTTTCCCAGTCACGAC-3' (SEQ ID NO. 21), and the downstream sequence was the downstream sequence of the corresponding oligo. For the positive clone, the bacteria were shaken, the plasmids were extracted (Axygene: AP-MN-P-250G), and the concentration was measured to be ready for use. The obtained mutated plasmids were named as mt-T7-sgRNA (SEQ ID NO. 11), re-U6-sgRNA (SEQ ID NO. 12) and re-T7-sgRNA (SEQ ID NO. 13).

### 1.2 In vitro transcription of sgRNA

A fragment containing sgRNA was amplified by using the constructed PUC57-T7sgRNA as a template, and the primers used were F: 5'-TCTCGCGCGTTTCGGTGATGACGG-3' (SEQ ID NO. 22), R: 5'-AAAAAAAGCACCGACTCGGTGCCACTTTTTC-3' (SEQ ID NO. 23). The amplification system was as follows: 25 µL of 2Xbuffer (Vazyme: P505), 1 µL of dNTP, 2 µL of F (10 pmol/µL), 2 µL of R (10 pmol/µL), 1 ng of template, 0.5 µL of DNA polymerase (Vazyme: P505), and ddH₂O complemented to 50µL. The amplified PCR product was purified by the following steps of adding 4 µL of RNAsecure (Life: AM7005) per 100 µL volume; the treatment was performed at 60°C for 15 minutes; adding three times volume of PCR-A (Axygen: AP-PCR-250G), performing column chromatography, centrifuging, centrifuging at a speed of 12,000 rpm for 1 minute, adding 500 µL of W2, and centrifuging for 1 minute; idling for 1 minute, and adding 20 µL of RNA-free water to perform elution.

The steps of transcribing by using an in vitro transcription kit (Ambion, Life Technologies, AM1354) were as follows. The reaction system was as follows: 1 µL of reaction buffer, 1 µL of enzyme mix, 1 µL of A, 1 µL of T, 1 µL of G, 1 µL of C, 800 ng of template, and H₂O complemented to 10 µL, The above system was mixed evenly and reacted at a temperature of 37°C for 5 hours. 1 µL of DNase was added and reacted at a temperature of 37°C for 15 minutes. The transcribed sgRNA was recovered by a recovery kit (Ambion, Life Technologies, AM1908) by the following steps: adding 90 µL of an Elution solution to the reaction volume of a product in the above step and transplanting the mixture to a 1.5 ml EP tube; adding 350 µL of a Binding solution to mix evenly; adding 250 µL of absolute ethanol to mix evenly; performing column chromatography; centrifuging at a speed of 10,000 rpm for 30 seconds, and pouring a waste solution out; adding 500 µL of a Washing solution, centrifuging at a speed of 10,000 rpm for 30 seconds, and pouring a waste solution out; idling for 1 minute; replacing a collection tube, and adding 100 µL of an Elution solution to perform elution; adding 10 µL of ammonium acetate (Ambion, Life Technologies, AM1908) to mix evenly; adding 275 µL of absolute ethanol to mix evenly; keeping stand at a temperature of -20°C for 30 minutes while preparing 70% ethanol and keeping stand at a temperature of -20°C; centrifuging at a speed of 13,000 rpm for 15 minutes at a temperature of 4°C; discarding a supernatant, and adding 500 µL of 70% ethanol; centrifuging for 5 minutes, drawing away a waste solution, and air-drying for 5 minutes; adding 20 µL of water to dissolve; and taking 1 µL and measuring the concentration.

### 1.3 Cell culture and electrotransfection

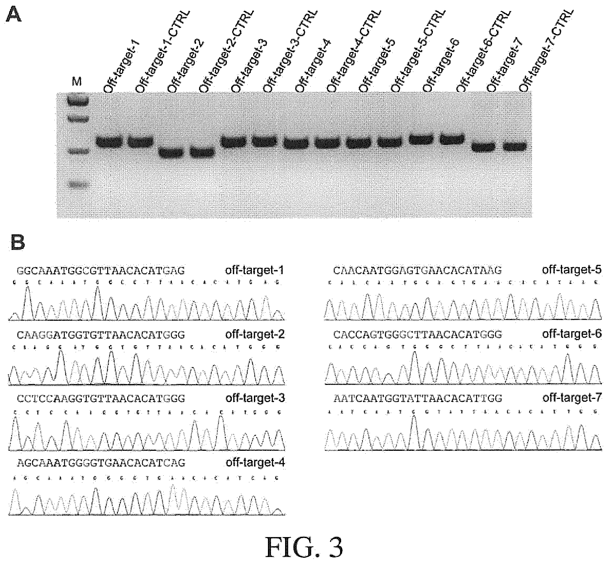

(1) Taking HEK293T cells (purchased from ATCC) as an example, the culture and transfection of eukaryotic cells were carried out in the present invention as follows: the HEK293T cells were inoculated and cultured in a DMEM high glucose culture solution (HyClone, SH30022.01B) added with 10% FBS, wherein penicillin (100 U/ml) and streptomycin (100 µg/ml) were contained.
(2) The medium was replaced with an antibiotic-free medium two hours before the transfection, and the transfection was carried out by using an LONZA transfection reagent (SF KIT) according to the instructions, and the cells were counted to be 1X10⁶. Cas9 (Sigma: ESPCAS9PRO-50UG), sgRNA and ssODN were mixed according to a mass of 3 µg, 1.5 µg and 3 µg. The electrotransfection procedure was performed by using DS150, and the electrotransfected cells were cultured in a 6 cm dish for two days.
(3) Single cells were sorted out from the cells by a flow sorter to be cultured. After two weeks, the genotype was identified by splitting decomposition. A lysate comprises the following components: 50 mM KCl, 1.5 mM MgCl₂, 10 mM Tris pH 8.0, 0.5% Nonidet P-40, 0.5% Tween 20, and 100µg/ml protease K. Cell lines that were homozygously mutated were selected and cultured on a large scale.
(4) For the mutated cell lines, 7 related off-target sites were identified by http://crispr.mit.edu/, https://crispr.cos.uni-heidelberg.de/, as shown in Table 1.
(5) The off-target sites were identified by designing corresponding primers, and the primer sequences were as shown in Table 3. The amplified PCR product was identified by T7EN1 and sequencing, and no off-target was found (Figure 3).

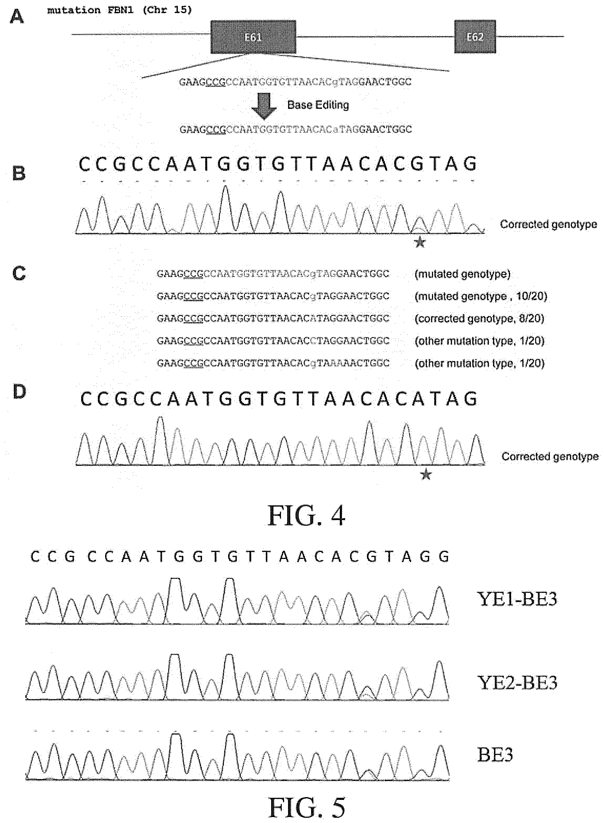

### 1.4 Repair of mutated cell lines (Figure 4, Figure 5)

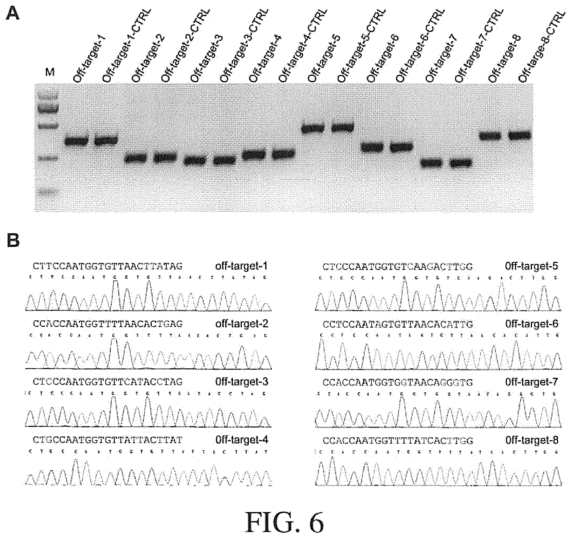

(1) The mutated cell lines were cultured and transfected in the present invention as follows: HEK293T cells were inoculated and cultured in a DMEM high glucose culture solution (HyClone, SH30022.01B) added with 10% FBS, which contains penicillin (100 U/ml) and streptomycin (100 µg/ml).
(2) The cells were distributed in a 6-pore plate before transfection, and transfected when the density reached 70%-80%.
(3) Transfection was exemplified by lipofection. 2 µg of BE3 or YE1-BE3 or YEE-BE3 plasmid was evenly mixed with 1 µg of re-sgRNApGL3-U6-sgRNA plasmid according to the operation manual of Lipofectamine™ 2000 Transfection Reagent (Invitrogen, 11668-019), and co-transfected into the cells per pore, the solution was replaced after 6-8 hours, and the cells were collected after 72 hours.
(4) The collected cells were firstly subjected to PCR product sequencing, and the repaired peak values were found (Figure 3). Further, the PCR product was subjected to TA cloning, and in the selected clones, the cloning efficiency of repair reached 50%.
(5) The off-target analysis of the repaired re-sgRNA was carried out. Similarly, 8 potential off-target sites were found, as shown in Table 2 and Figure 3. Similarly, the potential off-target sites were analyzed and detected (Table 3, Figure 6), and no off-target phenomenon was found.

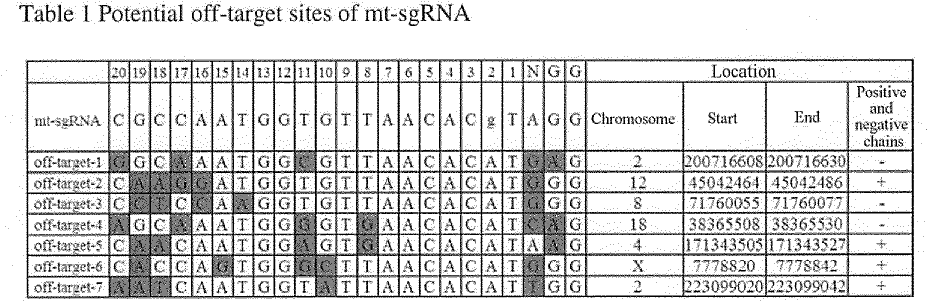

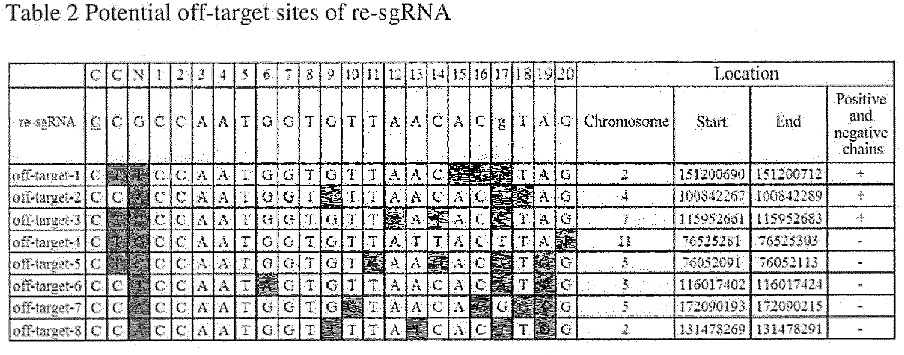

**Table 3 Primer sequences used in this project**

| Primer names | Sequences (5'- 3') | | Primer uses |
|---|---|---|---|
| FBN1-ON-200-F | ACTCACCAATGCAGGACGTA | SEQ ID NO.24 | Amplification of target sites |
| FBN1-ON-200-R | AGCTGCTTCATAGGGTCAGC | SEQ ID NO.25 | |
| FBN 1-OPN-676-F | GCTGAAGTCTCCACCCACC | SEQ ID NO.26 | |
| FBN1-ON-676-R | TGTCTCTCCTTGCCTTTTG | SEQ ID NO.27 | |
| FBN1 -mutation-off1-301 -F | TCAAGGGACAGGAGTAGGCA | SEQ ID NO.28 | Amplification of potential off-target sites |
| FBN1-mutation-off1-301-R | TTGGGGCAGGAGGTTTTGTT | SEQ ID NO.29 | |
| FBN1-mutation-off2-223-F | ATCTTAATCAGGGCCTTGA | SEQ ID NO.30 | |
| FBN 1-mutation-off2-223-R | GCCTTCATTCCATCAACTG | SEQ ID NO.31 | |
| FBN 1 -mutation-off3-296-F | CAGGTTCGTGTCGCAGTAGC | SEQ ID NO.32 | |
| FBN 1-mutation-off3-296-R | CTGTGTTGCCAGCACGAAA | SEQ ID NO.33 | |
| FBN 1-mutation-off4-282-F | TGGTAGTGGTTGGTGACACT | SEQ ID NO.34 | |
| FBN1-mutation-off4-282-R | CGTTACATTGGGAAGCGGAA | SEQ ID NO.35 | |
| FBN 1-mutation-off5-272-F | GGATTCAACATAGATTGGAA | SEQ ID NO.36 | |
| FBN 1-mutation-off5-272-R | CCCGTTTACACATTGCTA | SEQ ID NO.37 | |
| FBN1-mutation-off6-302-F | TTCTAGTAGGTGAAAAAGGG | SEQ ID NO.38 | |
| FBN 1-mutation-off6-302-R | TTGGACACCACATAGACAG | SEQ ID NO.39 | |
| FBN1-mutation-off7-252-F | TATTATTGCTAAACCGAAACCA | SEQ ID NO.40 | |
| FBN 1-mutation-off7-252-R | AGCCCCTCACCCACTCAT | SEQ ID NO.41 | |
| FBN 1-BE-OFF1-370-F | AGAGGCTTGCGAAGGACATC | SEQ ID NO.42 | Amplification of potential off-target sites |
| FBN1-BE-OFF1-370-R | ATTTGGTCTAGGGCAGAGGC | SEQ ID NO.43 | |
| FBN1-BE-OFF2-247-F | ATTATTCACAAGTTATGGTA | SEQ ID NO.44 | |
| FBN1-BE-OFF2-247-R | TAACCCTCTTCTTTGTAA | SEQ ID NO.45 | |
| FBN 1-BE-OFF3-235-F | AAGGGACTGTTTTTGTCCTGTCA | SEQ ID NO.46 | |
| FBN 1-BE-OFF3-235-R | GTGAAACCACCATGACATGAAGT | SEQ ID NO.47 | |
| FBN1-BE-OFF4-262-F | GTCATACTTGGCCAGGGTCC | SEQ ID NO.48 | |
| FBN 1-BE-OFF4-262-R | CCCACGTGAGCTGGCTAAAA | SEQ ID NO.49 | |
| FBN 1-BE-OFF5-448-F | TGATCAGCATGTGGAGCCTG | SEQ ID NO.50 | |
| FBN1-BE-OFF5-448-R | GAAGTCAGCCAGGAGCCATT | SEQ ID NO.51 | |
| FBN 1- BE-OFF6- 296- F | GAGTTAGGAGTGGGAAGG | SEQ ID NO.52 | |
| FBN1-BE-OFF6-296-R | ACAAAGGACAGTAATGAAGAG | SEQ ID NO.53 | |
| FBN1-BE-OFF7-208-F | TTTGCCTCCTTGATTCCCCC | SEQ ID NO.54 | |
| FBN1-BE-OFF7-208-R | GTGGATGGTGTGGAGGTGAG | SEQ ID NO.55 | |
| FBN1-BE-OFF8-370-F | CGATAAAGGGATCAGTCACTAA | SEQ ID NO.56 | |
| FBN 1-BE-OFF8-370-R | GCTCCAGGTCCACAAACAC | SEQ ID NO.57 | |

### Embodiment 2

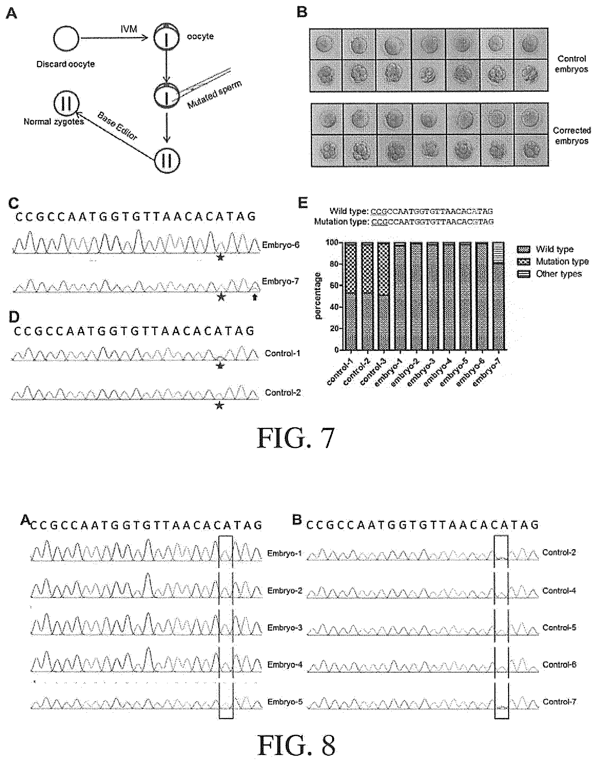

In this embodiment, the mutation was repaired by using a base editor in human embryos (Figure 7).

### 2.1 Plasmid construction

Near the mutation site, according to the characteristics of base editing, a repair re-sgRNA (SEQ ID NO. 3) was designed and oligos were synthesized, the upstream sequence was 5'-taggCTACGTGTTAACACCATTGG-3' (SEQ ID NO. 18), and the downstream sequence was 5'-aaacCCAATGGTGTTAACACGTAG-3' (SEQ ID NO. 19). The upstream and downstream sequences were annealed by a procedure (95°C, 5 min; 95°C - 85°C at - 2°C/s; 85°C - 25°C at - 0.1°C/s; hold at 4°C), and ligated to a PUC57-T7sgRNA vector linearized with BsaI (NEB: R0539L). The linearization system and procedure were as above. The ligation system was as follows: 1 µL of T4 ligation buffer (NEB: M0202L), 20 ng of linearized vector, 5 µL of annealed oligo fragment (10 µM), 0.5 µL of T4 ligase (NEB: M0202L), and ddH₂O complemented to 10 µL. Ligation was carried out overnight at a temperature of 16°C. The ligated vector was subjected to transformation, bacterium selection and identification, the identification primer was the upstream sequence: 5'-CGCCAGGGTTTTCCCAGTCACGAC-3' (SEQ ID NO. 21), and the downstream sequence was the downstream sequence of the corresponding oligo. For the positive clone, the bacteria were shaken, the plasmids were extracted (Axygene: AP-MN-P-250G), and the concentration was measured to be ready for use..

### 2.2 In vitro transcription of sgRNA

A fragment containing sgRNA was amplified by using the constructed PUC57-T7sgRNA as a template, and the primers used were F: 5'-TCTCGCGCGTTTCGGTGATGACGG-3' (SEQ ID NO. 22), R: 5'-AAAAAAAGCACCGACTCGGTGCCACTTTTTC-3' (SEQ ID NO. 23). The amplification system was as follows: 25 µL of 2Xbuffer (Vazyme: P505), 1 µL of dNTP, 2 µL of F (10 pmol/µL), 2 µL of R (10 pmol/µL), 1 µg of template, 0.5 µL of DNA polymerase (Vazyme: P505), and ddH₂O complemented to 50µL. The amplified PCR product was purified by the following steps of adding 4 µL of RNAsecure (Life: AM7005) per 100 µL volume; the treatment was performed at 60°C for 15 minutes; adding three times volume of PCR-A (Axygen: AP-PCR-250G) to perform column chromatography, centrifuging, centrifuging at a speed of 12,000 rpm for 1 minute, adding 500 µL of W2, and centrifuging for 1 minute; idling for 1 minute, and adding 20 µL of RNAase-free water to perform elution.

The steps of transcribing by using an in vitro transcription kit (Ambion, Life Technologies, AM1354) were as follows. The reaction system was as follows: 1 µL of reaction buffer, 1 µL of enzyme mix, 1 µL of A, 1 µL of T, 1 µL of G, 1 µL of C, 800 ng of template, and H₂O complemented to 10 µL. The above system was mixed evenly and reacted at a temperature of 37°C for 5 hours. 1 µL of DNase was added and reacted at a temperature of 37°C for 15 minutes. The transcribed sgRNA was recovered by a recovery kit (Ambion, Life Technologies, AM1908) by the following steps: adding 90 µL of an Elution solution to the reaction volume of a product in the above step and transplanting to a 1.5 ml EP tube; adding 350 µL of a Binding solution to mix evenly; adding 250 µL of absolute ethanol to mix evenly; performing column chromatography; centrifuging at a speed of 10,000 rpm for 30 seconds, and pouring a waste solution out; adding 500 µL of a Washing solution, centrifuging at a speed of 10,000 rpm for 30 seconds, and pouring a waste solution out; idling for 1 minute; replacing a collection tube, and adding 100 µL of an Elution solution; adding 10 µL of ammonium acetate (Ambion, Life Technologies, AM1908) to mix evenly; adding 275 µL of absolute ethanol to mix evenly; keeping stand at a temperature of -20°C for 30 minutes while preparing 70% ethanol and keeping stand at a temperature of -20°C; centrifuging at a speed of 13,000 rpm for 15 minutes at a temperature of 4°C; discarding a supernatant, and adding 500 µL of 70% ethanol; centrifuging for 5 minutes, drawing away a waste solution, and air-drying for 5 minutes; adding 20 µL of water to dissolve; and taking 1 µL and measuring the concentration.

### 2.3 In vitro transcription of BE3

BE3 enzyme digestion and recovery. This step was to linearize the plasmid BE3. The system was as follows: 10 µg of BE3/YE1-BE3/YE2-BE3/YEE-BE3, 10 µL of buffer I (NEB: R0539L), 4 µL of BbsI (NEB: R0539L), and H₂O complemented to 100 µL. After mixing evenly, the enzyme digestion was carried out overnight at a temperature of 37°C.

Recovery of linearized plasmid. 4 µL of RNAsecure (Life: AM7005) was added to an enzyme-digested product, and reacted at a temperature of 60°C for 10 minutes; the remaining steps were carried out by using a recovery kit (QIAGEN: 28004), 5 times volume of buffer PB was added and column chromatography was performed; 750 µL of buffer PE was added for centrifugation; the idling was carried out for 1 minute; 10 µL of water was used for elution and the concentration was measured.

In vitro transcription. According to the requirements of a kit (Invitrogen: AM1345), relevant reagents were sequentially added to the system: 1 µg of linearized vector, 10 µL of 2XNTP/ARCA, water complemented to 20 µL, 2 µL of T7 ezyme mix, and 2 µL of 10x reaction buffer. After mixing, the reaction was carried out at a temperature of 37°C for 2 hours. 1 µL of DNasea was added to react for 15 minutes.

Tailing. The transcription product was subjected to a tailing treatment to ensure the stability of the transcribed mRNA. The specific system was as follows: 20 µL of reaction product, 36 µL of H₂O, 20 µL of 5xE-PAP buffer, 10 µL of 25 mM MnCl₂, 10 µL of ATP solution, and 4 µL of PEP. The reaction system was mixed evenly and then reacted at a temperature of 37°C for 30 minutes.

Recovery. The recovery was carried out by using a recovery kit (QIAGEN: 74104). The steps were as follows: adding 350 µL of buffer RLT to the reaction product in the above step; adding 250 µL of absolute ethanol, performing column chromatography and centrifuging; adding 500 µL of RPE, centrifuging, adding 500 µL of RPE, and centrifuging; idling, and adding 30 µL of water to elute. After the concentration was measured, it was stored at a temperature -80°C.

### 2.4 Obtaining of mutated embryos

All embryo operations were performed at the Reproductive Medicine Center of the Third Affiliated Hospital of Guangzhou Medical University. The experiment has been approved by the ethics committee of the hospital, and patients who donated eggs and sperms have signed an informed consent. In this experiment, *FBN1^{T7498C}* heterozygous mutation patients were obtained and their genotypes were identified by blood and semen in the experiment (Figure 1). The used eggs were all immature eggs of patients with reproductive disorders and matured by being cultured in vitro. The mutated sperms were bound to the eggs by ICSI to obtain mutated zygotes.

### 2.5 Embryo repair operations

When the zygotes were observed to be in the 2PN stage, the base editing mRNA with volume of approximately 0.2 µL, BE3 used in this embodiment, and the sgRNA cytoplasm used for repair were injected into the zygotes by micromanipulation, and the concentrations of BE3 and sgRNA were 100 ng/µL and 50 ng/µL respectively. The treated embryonic cells were further cultured in a tri-gas incubator for three days (Figure 7).

### 2.6 Embryo amplification and identification

Single-cell amplification of the collected embryos was performed by using a reagent Vazyme, N601-01. The amplified genome was diluted 100 times to amplify target fragments to detect the efficiency of mutation (Figure 8). Primers for amplifying the target fragments were shown in Table 3.

### 2.7 Determination of editing efficiency by deep sequencing

To further confirm the editing efficiency, the target fragments were detected by means of PE150 high-throughput sequencing. Three control embryos and all seven repaired embryos were selected. It was found that the three embryos in a control group were of heterozygous genotypes, and the seven repaired embryos were all of normal genotypes, which proves that the base editor can efficiently implement the target site repair.

### 2.8 Detection of off-target sites of embryos

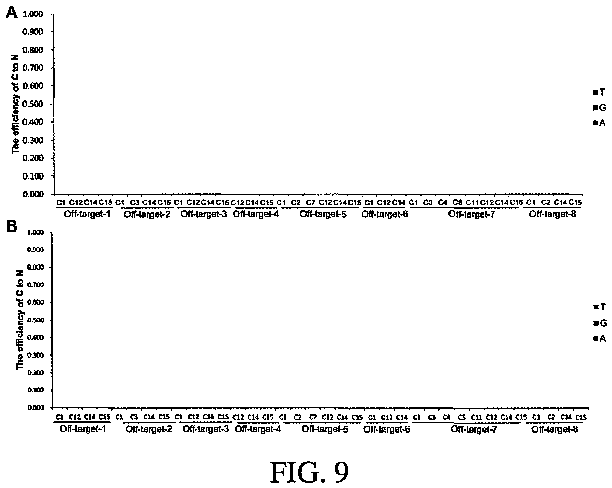

To ensure the safety of the base editor, the off-target detection of three control embryos and all repaired embryos was performed. The results showed (Figure 9) that no obvious off-target phenomenon was found in the repaired embryos.

## Claims

1. A kit for efficiently repairing an *FBN1^{T7498C}* mutation, wherein the kit comprises a base editor and a repair re-sgRNA directed to an *FBN1^{T7498C}* site.

2. The kit for efficiently repairing an *FBN1^{T7498C}* mutation according to claim 1, wherein the base editor is one of BE3, YE1-BE3, YE2-BE3 or YEE-BE3.

3. The kit for efficiently repairing an *FBN1^{T7498C}* mutation according to claim 1, wherein the sequence of the repair re-sgRNA directed to the *FBN1^{T7498C}* site is SEQ ID NO. 3.

4. A combination for making a mutation and repairing a mutation, wherein the combination comprises at least one of a mutated mt-sgRNA designed according to an *FBN1^{T7498C}* site and a corresponding mutated ssODN, a repair re-sgRNA directed to the *FBN1^{T7498C}* site, and a base editor.

5. A method for repairing a mutation by using base editing, wherein the method comprises: in an *FBN1^{T7498C}*-containing mutated cell, using a repair re-sgRNA directed to an *FBN1^{T7498C}* site to guide a base editor to a mutation site to perform base editing repair and collect transfected cells.

6. The method for repairing a mutation by using base editing according to claim 5, wherein the *FBN1^{T7498C}*-containing mutated cell is an HEK293T cell.

7. The method for repairing a mutation by using base editing according to claim 5, wherein a method for constructing the *FBN1^{T7498C}*-containing mutated cell comprises: designing a mutated mt-sgRNA and a corresponding mutated ssODN according to the *FBN1^{T7498C}* site; constructing an expression vector of mt-sgRNA, allowing a Cas9 protein and the transcribed mt-sgRNA in vitro to form an RNP to be bound to ssODN and electrotransfect HEK293T cells, and performing flow sorting on single cells and identifying an *FBN1^{T7498C}*-containing mutated cell line.

8. The method for repairing a mutation by using base editing according to claim 5, wherein the repair re-sgRNA directed to the *FBN1^{T7498C}* site is obtained by designing the repair re-sgRNA according to the *FBN1^{T7498C}* site and constructing a U6 started and/or T7 started expression vector.

9. The method for repairing a mutation by using base editing according to claim 7, wherein the sequence of the mt-sgRNA is SEQ ID NO. 1, the sequence of ssODN is SEQ ID NO. 2, and the sequence of re-sgRNA is SEQ ID NO.3.
